# EUROPEAN PATENT APPLICATION

(11) **EP 2 332 898 A1**
(43) Date of publication of application: **15.06.2011**
(21) Application number: 09178735.8
(22) Date of filing: 10.12.2009
(51) Int. Cl.: C07C 50/28, A61Q 19/08, A61P 39/06

(54) **Benzoquinone-based antioxidants**

(71) Applicant: Universität des Saarlandes, 66123 Saarbrücken (DE)
(72) Inventor: Gulaboski, Rubin, 2000 Stip (MK); Bogeski, Ivan, 66424 Homburg (DE); Kappl, Reinhard, 66424 Homburg (DE); Hoth, Markus, Prof., 66424 Homburg (DE)
(74) Representative: von Kreisler Selting Werner

(57) **Abstract**

The invention relates to a new type of highly polar, at least double-negatively charged redox-active benzoquinones, notably derived from Coenzyme Q0, Q1 and dimethoxy benzoquinones, its production and use. The production of these benzoquinones comprises the reaction of dimethoxy benzoquinones with highly alkaline aqueous solutions. The benzoquinones of the invention have various potential applications in pharmaceutical and cosmetic industry (namely as antioxidants, anti-ageing agents and the like), in organic synthesis, metal complexations, metal separations, potentiometric titrations, interfacial catalysis, and ion-transfer reactions across biological membranes.

## Description

The invention relates to a new type of highly polar, at least double-negatively charged redox-active benzoquinones, notably derived from Coenzyme Q0, Q1 and dimethoxy benzoquinones, its production and use. The production of these benzoquinones comprises the reaction of dimethoxy benzoquinones with highly alkaline aqueous solutions. The benzoquinones of the invention have various potential applications in pharmaceutical and cosmetic industry (namely as antioxidants, anti-ageing agents and the like), in organic synthesis, metal complexations, metal separations, potentiometric titrations, interfacial catalysis, and ion-transfer reactions across biological membranes.

### Background of the Invention

Among the redox active compounds involved in the electron/proton transfer reactions taking place in the living systems, the quinones are probably the most studied examples. Molecules with quinoid structure constitute one of the most interesting classes of redox compounds in living systems. Quinones are widely distributed in nature and their basic structure is featuring important classes of compounds like Vitamin K1, various cofactors and coenzymes (various compounds of the CoQ10-like family), flavonoids and many more (Morron, R., Biochemistry of Quinones, New York-London: Academic Press Inc. (1965); Rahman, A., Bioactive Natural Products, ed. Rahman: Elsevier (2002); Swenton J., Chemistry of Quinones, New York: John Wiley (1988)). The electron and proton transfer functions of many quinone-like compounds are of fundamental importance to all living forms. Next to their irreplaceable role in the electron/proton transfer chain in mitochondria (Coenzyme Q10, plastoquinone), many of the quinones function as effective radical scavengers and antioxidants, to protect the living cells from oxidative damages. Due to their effective antioxidizing activities, some of the quinones are used as therapeutic and anti-tumor agents. The application of the quinones as reducing/oxidizing reagents attains remarkable attention in various fields of organic chemistry, pharmacy, medicine, physiology and biochemistry. Given all the attention to their recognized and well-established functions in the living organisms and in everyday biochemistry, there are significant parts of their redox chemistry and their functions that are still not resolved. While the mechanistic pathway of electron transfer in two successive one-electron steps for quinones in organic (aprotic) media seems to be unanimously defined and accepted (Jucker, E. Progress in Drug Research, Basel: Birkhauser Verlag (1995)), their electrochemistry in water solutions is still a subject of many disputes and controversies (Srinivasan, S; Chizmadzhev, Y.A., Bioelectrochemistry, Springer (1985); Quan, M. et al., J. Am. Chem. Soc. 129(42:p.12847-56 (2007); Bartlet, P.N., Bioelectrochemistry; fundamentals, experimental techniques, and applications, Wiley (2008)). The discrepancies arise, because in some cases the redox chemistry of quinones in water solutions can be well explained by a coupled 2e⁻/2H⁺ reaction with a conversion between quinone and hydroquinone. However, many quinones undergo very complicated redox pathway in water solutions, so that their electrochemical behavior cannot be always described by the 2e⁻/2H⁺ redox reaction scheme. This complex behavior of the quinones is certainly closely tied to the nature of the substituents on the aromatic ring. The chemistry of quinones is largely dependent on the substituents being either on the quinonic or on adjacent rings, and this is reflected in their chemical reactivity. The steric constraints and the electron density distribution in many substituted quinone systems frequently affect the electrochemical properties and their reactivity in unpredictable fashion. Very recently, in comprehensive electrochemical studies, the authors presented strong evidences that the electrochemical transformation of quinones in unbuffered water solutions produces quinone dianions (Q²⁻), which are stabilized by strong hydrogen bonding with water (Quan, M. et al., J. Am. Chem. Soc. 129(42:p.12847-56 (2007)). Previously, the existence of Q²⁻ species that are energetically stabilized via the hydrogen bonding with the water molecules, has also been predicted from theoretical *ab initio* calculations (Manojkumar, T.K. et al., The Journal of Chemical Physics 118(19):p.8681-8686 (2003)). These recent evidences for the presence of stable anionic forms of quinones in water can certainly help to reveal the function, the mechanistic pathways and reactivity of quinones in aqueous media.

### Short Description of the Invention

It was now found that substituted dimethoxy benzoquinones derivatives and Coenzyme Q family members that - when dissolved in a strong alkaline aqueous solution such as NaOH or KOH - react with the aqueous alkaline solution giving as products new highly polar benzoquinone derivatives that show remarkable chemical and physical features, and possess pronounced antioxidant and metal-chelating abilities. Electrochemical, spectrophotometrical, electron paramagnetic resonance (EPR), Nuclear magnetic resonance (NMR) techniques were used to characterize the structure and the properties of the polar benzoquinone derivatives generated by this reaction. The invention thus provides
(1) a di-, tri- or tetraoxy-para-benzoquinone compound having the formula (I) 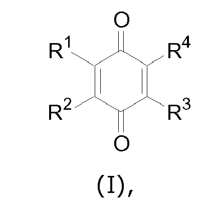 wherein at least two of R¹ to R⁴ are O⁻ residues and the remaining are independently selected from H, alkyl, alkenyl and alkoxy, or, if adjacent, may form a carbocyclic or heterocyclic ring, which may be saturated, unsaturated or aromatic, together with the two carbon atoms of the benzoqinone ring, or a salt thereof;
(2) a preferred embodiment of (1) above, wherein the compound is a dioxy-para-benzoquinone compound having a formula (IIa), (IIb) or (IIc) 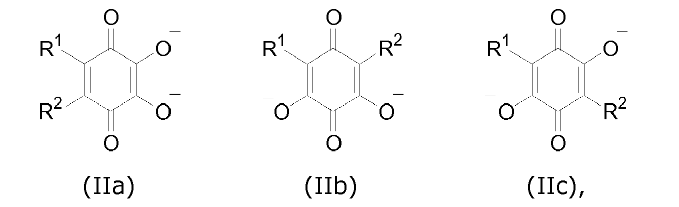 wherein R¹ and R² are independently selected from H, alkyl, alkenyl and alkoxy, or in formula (IIa) R¹ and R², together with the two carbon atoms of the benzoqinone ring, may form a carbocyclic or heterocyclic ring which may be saturated, unsaturated or aromatic, or a salt thereof;
(3) a preferred embodiment of (2) above, wherein the compound is a 2,3-dioxy-para-benzoquinone compound having a formula (IIa) or a salt thereof as defined in (2) above;
(4) a method for preparing the di-, tri- or tetraoxy-para-benzoquinone compound having the formula (1), (IIa), (IIb) or (IIc) as defined in (1) to (3) above, which comprises reacting a starting benzoquinone derivative of the formula (I) 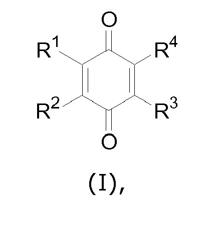 wherein two out of R¹ to R⁴ are independently selected from H, alkyl, alkenyl and alkoxy, or, if adjacent, may form a carbocyclic or heterocyclic ring, which may be saturated, unsaturated or aromatic, together with the two carbon atoms of the benzoqinone ring, and the two others are independently selected from H and alkoxy, with a concentrated aqueous alkaline solution;
(5) a preferred embodiment of (4) above for the preparation of dioxy-parabenzoquinones, which comprises reacting a compound of formula (I), wherein R¹ and R² are as defined in (2) above, and R³ and R⁴ are independently selected from H and alkoxy, respectively, with the concentrated aqueous alkaline solution;
(6) a composition, reduction agent or stabilizer comprising a compound of (1) to (3) above or a mixture thereof;
(7) the use of the compound of (1) to (3) above for preparing a medicament having antioxidant properties; and
(8) the use of the compound of (1) to (3) above as antioxidant, anti-ageing agent, in organic synthesis, for metal complexations, metal separations, potentiometric titrations, interfacial catalysis, and ion-transfer reactions across biological membranes.

The di-, tri- or tetraoxy-para-benzoquinone compounds of (1) above, the dioxy-para-benzoquinone compounds of (2) above and the 2,3-dioxy-para-benzoquinone compounds of (3) above, hereinafter shortly referred to as "compounds of the invention", have much stronger antioxidant potentials than the electrochemically reduced forms of their native ones and can form strong complexes with the earth-alkaline cations (Ca²⁺, Ba²⁺, and Sr²⁺) but weak ones with Mg²⁺. The compounds of the invention have various potential applications in medical and pharmaceutical industry (antioxidant, anti-ageing), organic synthesis, metal complexations, metal separations, potentiometric titrations, interfacial catalysis, and ion-transfer reactions across the biological membranes.

### Short Description of the Figures

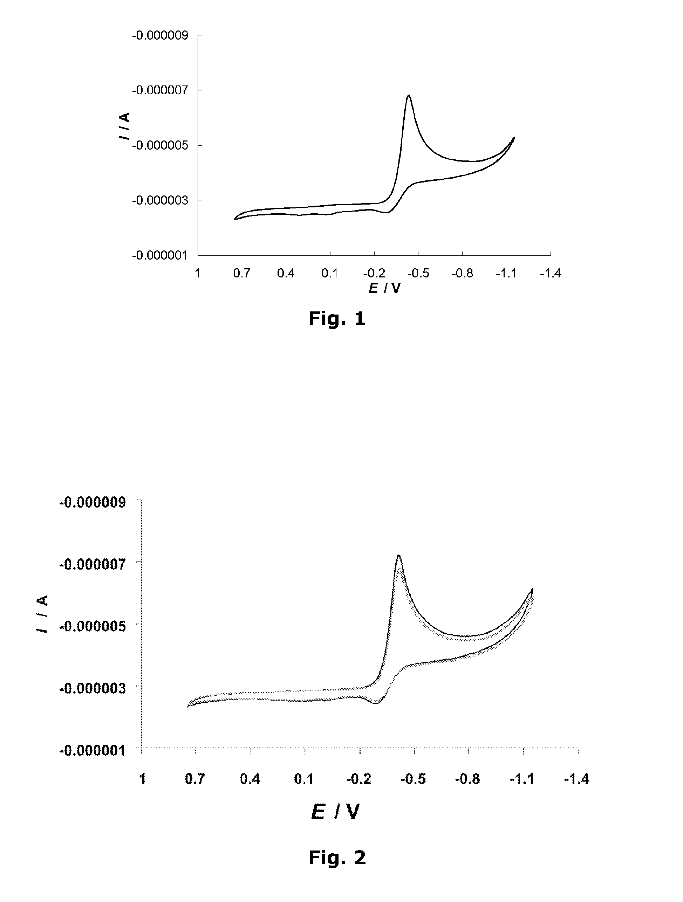

Fig. 1: Cyclic voltammograms of native (yellow form) CoQ1 in an aqueous solution with a pH of 7.40. A stock solution of CoQ1 was prepared by dissolving it in 0.1 mol/l KCl. Scan rate = 30 mV/s; c(CoQ1) = 50 µmol/l.
Fig. 2: Cyclic voltammograms showing insensitivity of native CoQ1 to Ca²⁺ ions in a pH of 7.40. c(Ca²⁺)/mmol●l⁻¹ = 1 (black curve), 10 (gray curve) and 100 (light gray curve). A stock solution of CoQ1 was prepared by dissolving it in 0.1 mol/l KCl; scan rate = 30 mV/s; c(CoQ1) = 50 µmol/l.

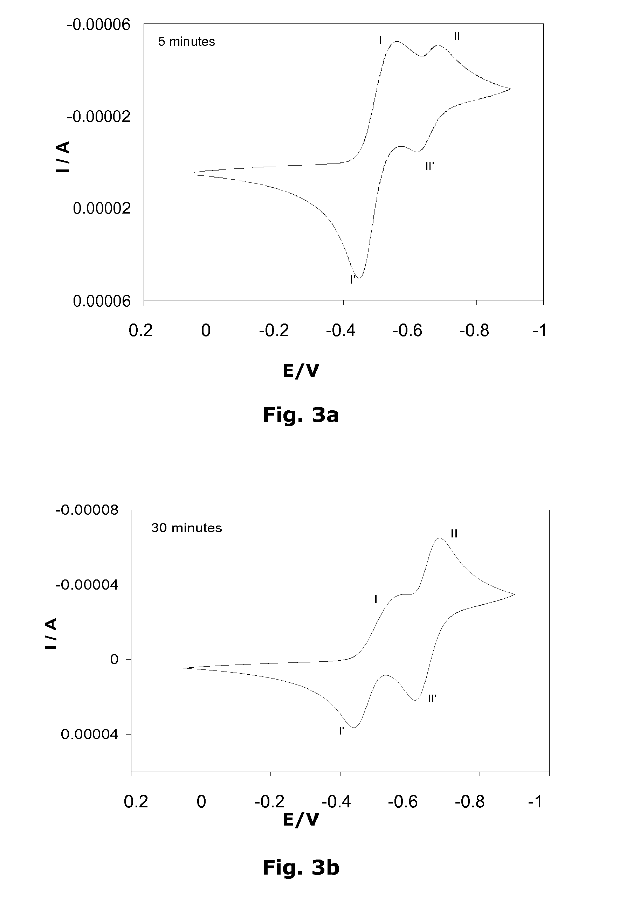

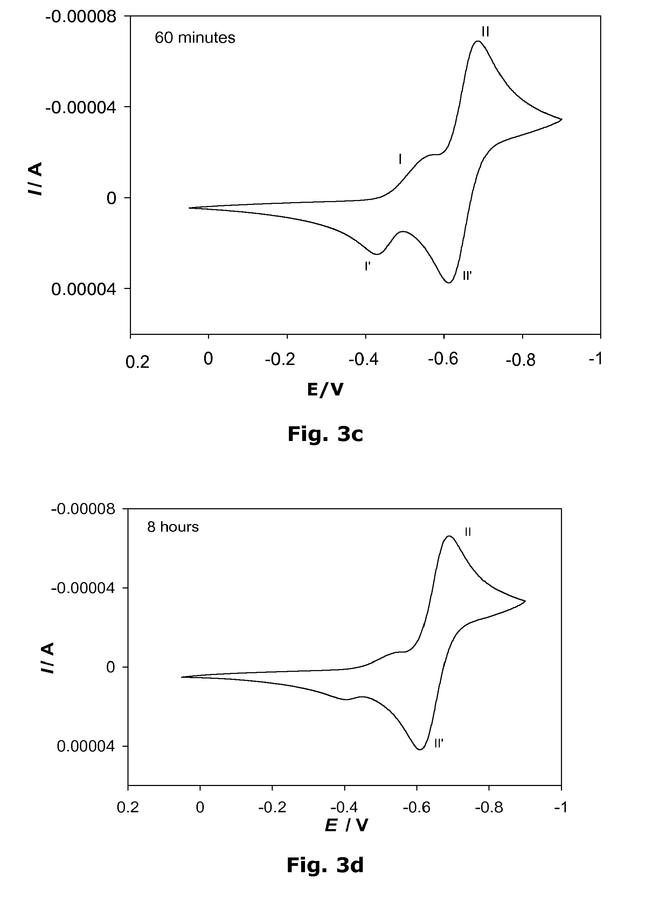

Fig. 3: Cyclic voltammograms of CoQ1 recorded in 1 mol/l NaOH. Scans recorded after 5 min (a), 30 minutes (b), 1 h (c) and 8 h (d); scan rate = 30 mV/s; c(CoQ1) = 0.5 mmol/l.

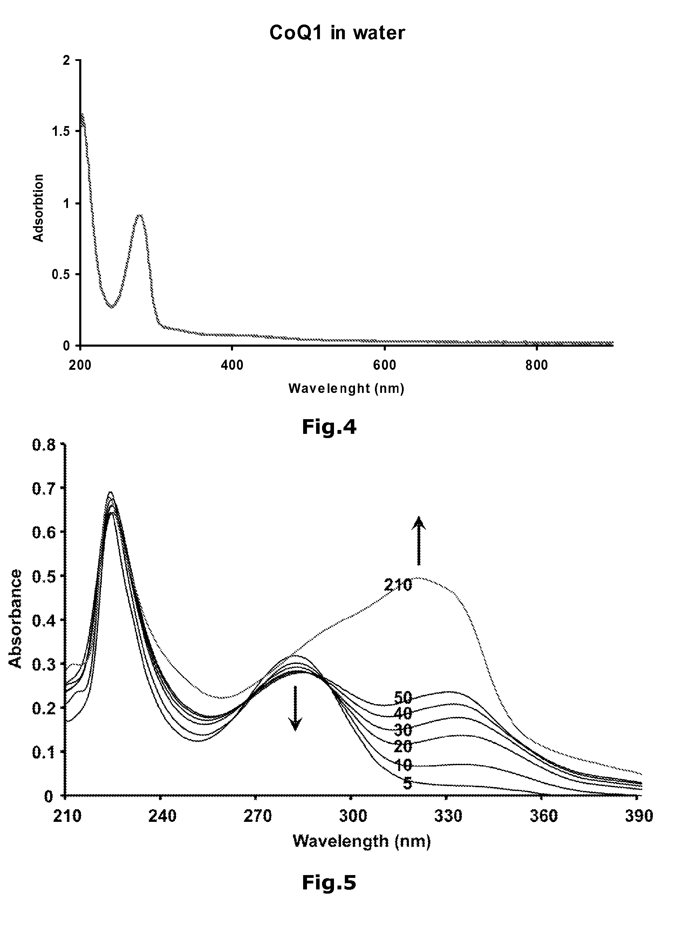

Fig. 4: UV-VIS spectrum of 10 µmol/l CoQ1 (the native yellow form) dissolved in water solution having pH of 7.40.
Fig. 5: UV-VIS spectra of 10 µmol/l CoQ1 recorded in kinetic mode in 1 mol/l NaOH. The numbers displayed are the reaction times (in min) between CoQ1 and NaOH.

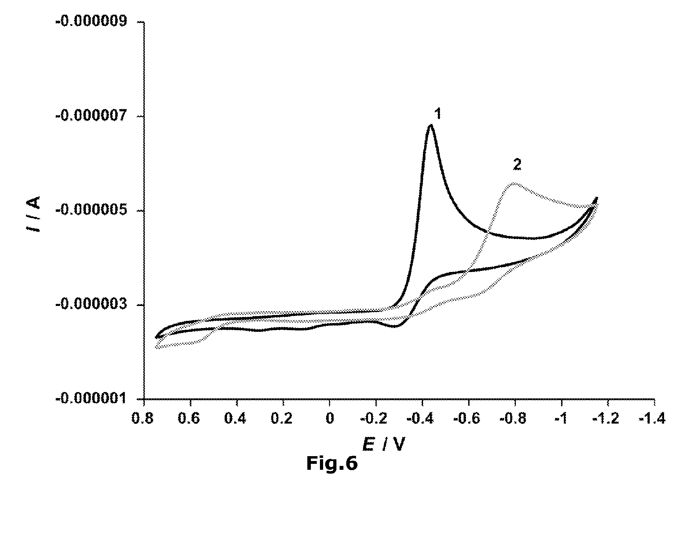

Fig. 6: Curve 1-Cyclic voltammograms of native CoQ1 (the "yellow form") dissolved directly in neutral media; Curve 2-Cyclic voltammograms of synthesized "purple form" of CoQ1. In this case (curve 2), CoQ1 was initially in contact with NaOH for 6 h. In both cases, the final pH was adjusted to 7.40; c(CoQ1) = 50 pmol/l; scan rate = 30 mV/s.

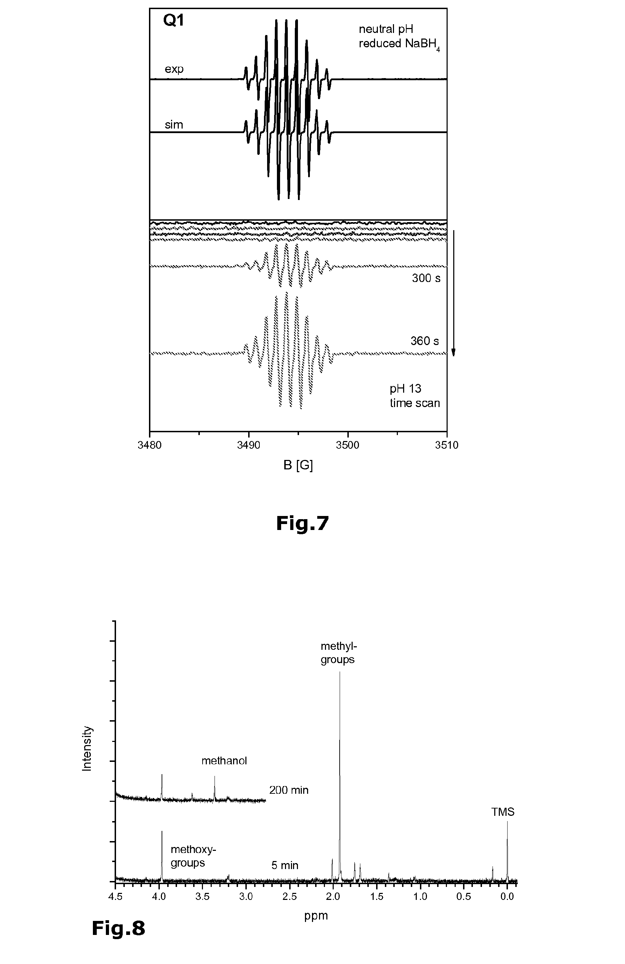

Fig. 7: EPR spectra of Q1.
Fig. 8: NMR spectra of 1 mg/ml CoQ1 dissolved in 1 mol/l NaOD for different times, and re-titrated to a pD of 7.0 with DNO₃.

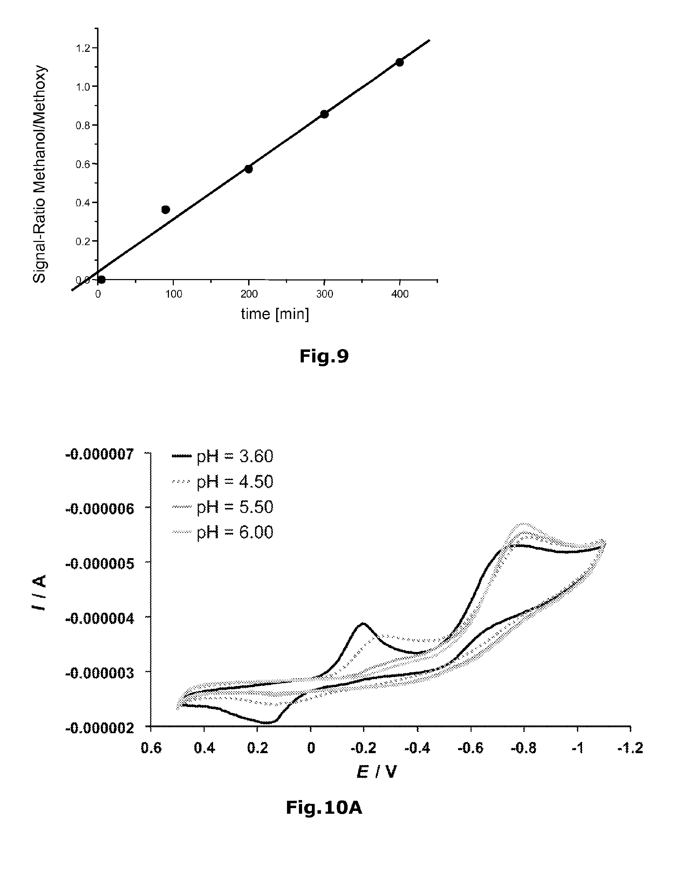

Fig. 9: Ratio of the relative integrals from the signal of methanol and methoxy groups as a function of time. Tetramethyl Silane was internal standard. This ratio is measured from the NMR spectra of 1 mg/ml CoQ1 dissolved in 1 mol/l NaOD for different times, and re-titrated to a pD of 7.0 with DNO₃ (Figure 8).

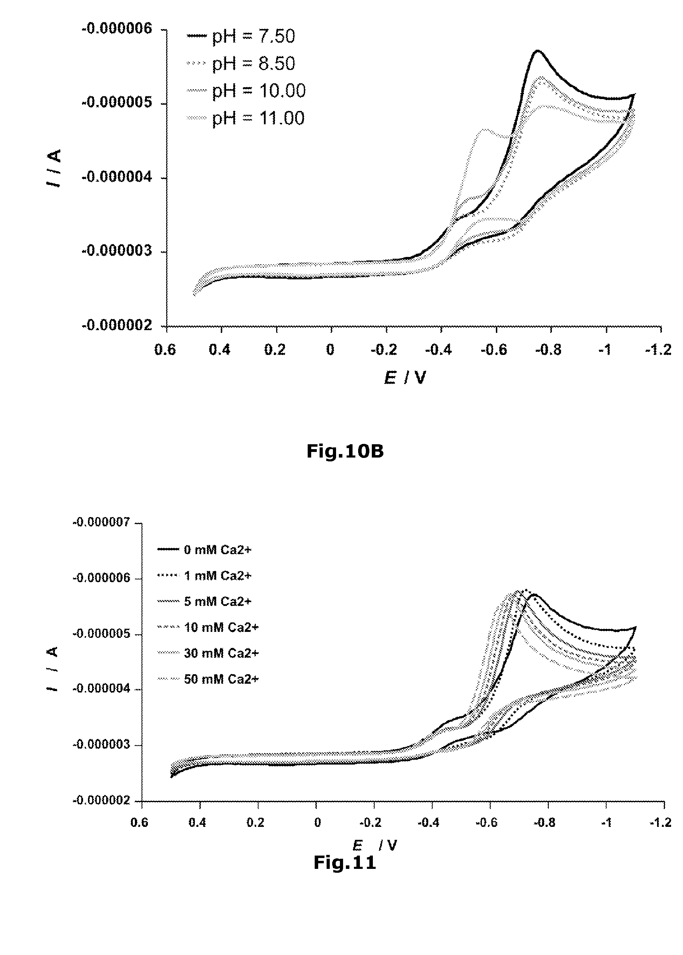

Fig. 10: Cyclic voltammograms of 50 µM CoQ1 (initially dissolved in 1 mol/l NaOH for 60 min) as a function of pH; scan rate = 30 mV/s.
Fig. 11: Cyclic voltammograms showing the Ca²⁺-sensitivity of the redox process of O-demethylated CoQ1. In this case, CoQ1 was initially dissolved in 1 mol/l NaOH for 6 hours, and the final pH was adjusted to 7.40 by making titration with 2 mol/l nitric acid, the concentration of CoQ1 was 50 pmol/l.

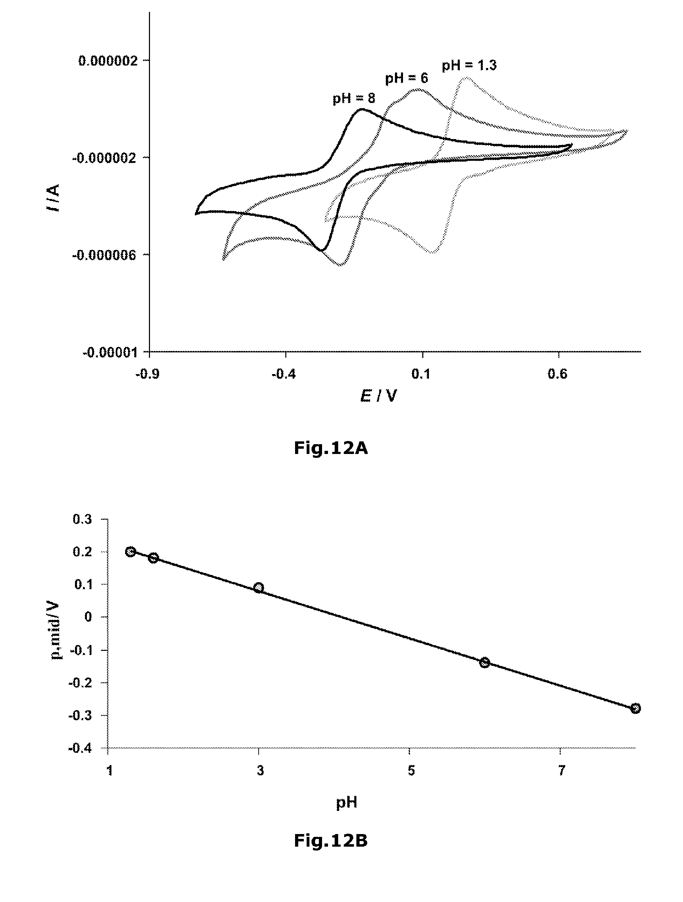

Fig. 12 A: Cyclic voltammograms of 500 µmol/l native 2,6-dimethoxy 1,4-benzoquinone recorded in buffers having pH ranging from 1.3 to 8.0. An increase of the concentration of H⁺ causes shift of the mid-peak potentials of the cyclic voltammograms toward more positive values. B: Plot of the dependence of the mid-peak potentials vs. pH; scan rate = 30 mV/s.

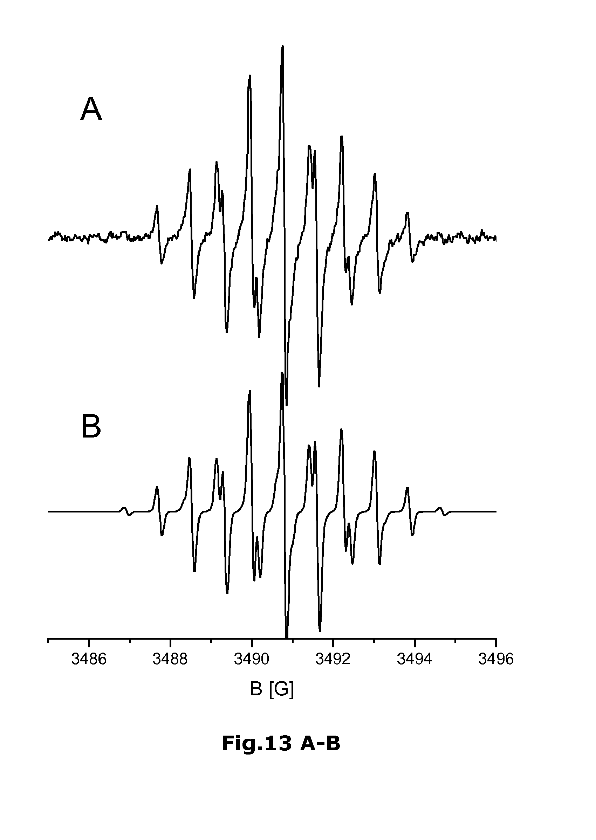

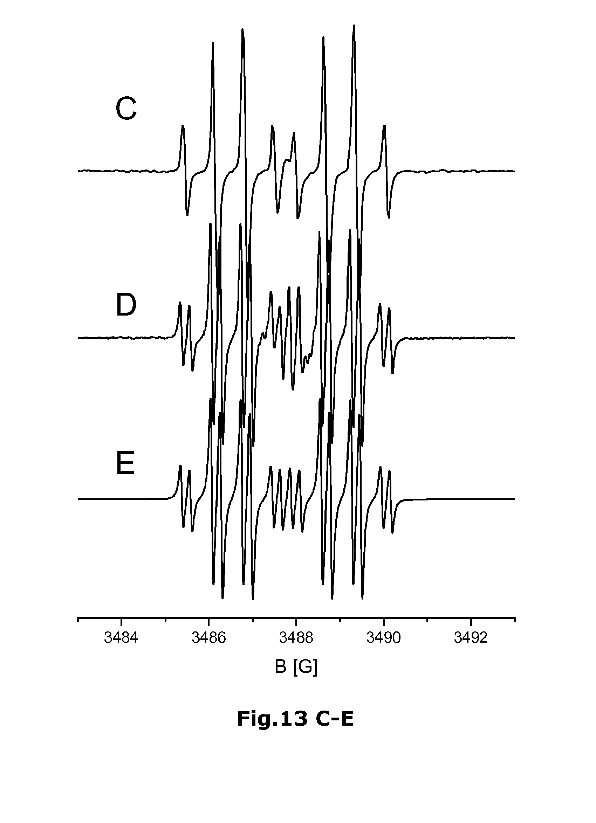

Fig. 13: Comparison of EPR spectra of 2,6-dimethoxy benzoquinone in neutral solution after reduction with sodium borhydrid (A, B) and in strongly alkaline conditions (C, D, E). Spectra A and C are obtained in deuterated solvent, D in H₂O solution. Spectra B and E are simulations showing very good agreement with the experiments. From the simulation parameters the structure of the radical can be derived.

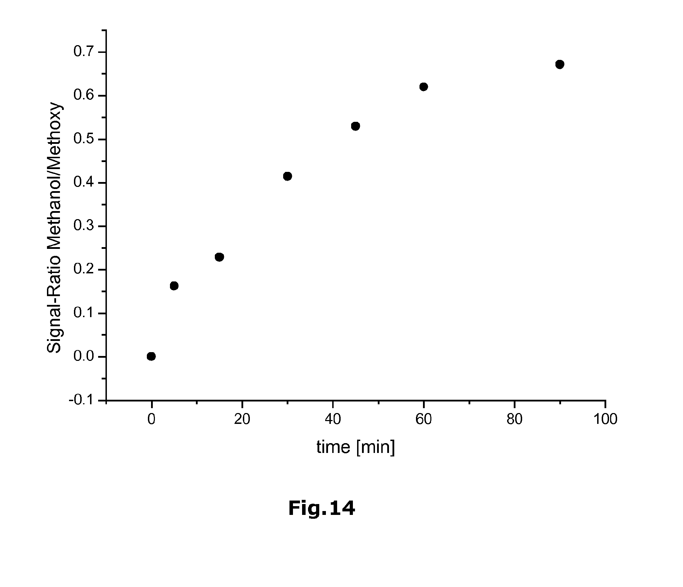

Fig. 14: Ratio of the relative integrals from the NMR signal of methanol and methoxy groups as a function of time. Tetramethyl Silane was internal standard. This ratio is measured from the NMR spectra of 1mM 2,6-dimethoxy benzoquinone dissolved in 1 mol/l NaOD for different times, and re-titrated to a pD of 7.0 with DNO₃.

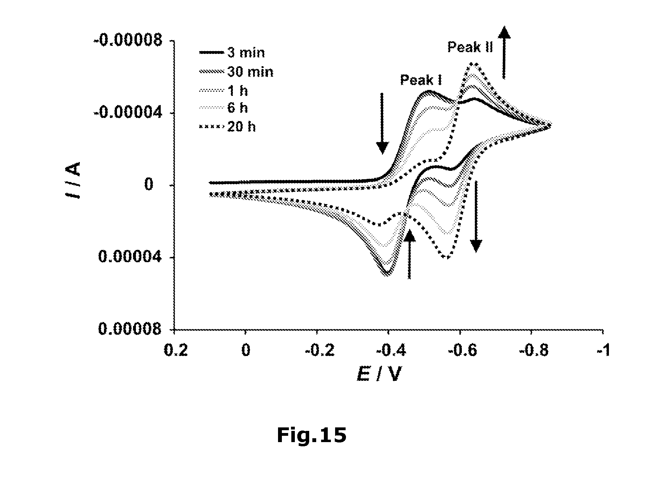

Fig. 15: Consecutive cyclic voltammograms of 2 mmol/l 2,6-dimethoxy 1,4-benzoquinone dissolved in alkaline water solution of 0.1 mol/l NaOH, recorded after different reaction times; scan rate = 30 mV/s.

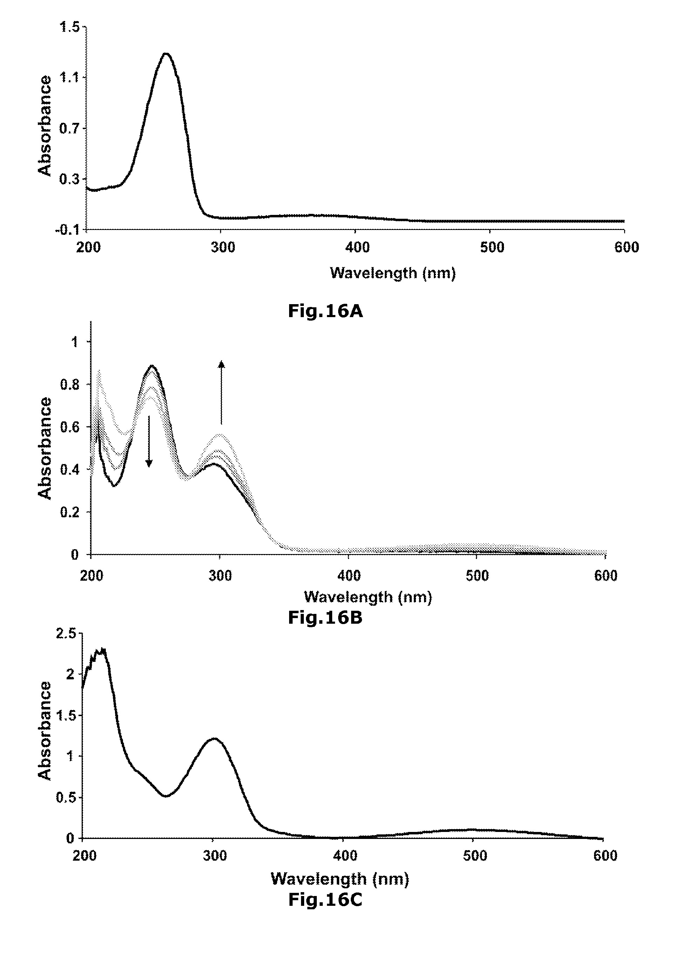

Fig. 16 A: UV-VIS spectra of 50 µmol/l 2,6-dimethoxy 1,4-benzoquinone (yellow form) in water. B: UV-VIS spectra of 50 µmol/l 2,6-dimethoxy 1,4-benzoquinone dissolved in 1 mol/l NaOH, recorded in kinetic mode after 5 min, 20 min, 60 min and 3 h (from black to light gray). C: UV-VIS spectrum of 2,6-dimethoxy 1,4-benzoquinone recorded in 1 mol/lNaOH after period of 24 h.

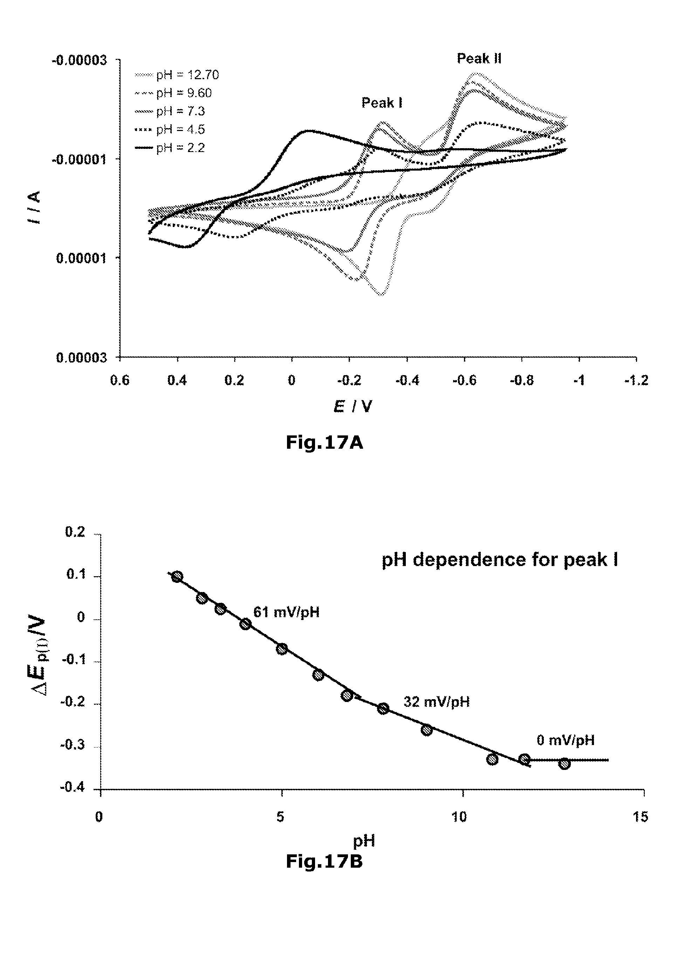

Fig. 17 A: Cyclic voltammograms of 1 mmol/l 2,6-dimethoxy 1,4-benzoquinone as a function of pH. In this case, 2,6-dimetoxy-1,4-benzoquinone was initially dissolved in 0.1 mol/l NaOH for 1 h, while pH was subsequently adjusted to the desired values. While the position of Peak I (that originates from the native form of 2,6-dimethoxy 1,4-benzoquinone) is sensitive to pH, the position of Peak II (that originates from the synthesized product from Example 4) is insensitive to pH. B: Plot of the mid-peak potentials of the Peak I (the yellow form of 2,6-dimethoxy 1,4-benzoquinone) as a function of pH.

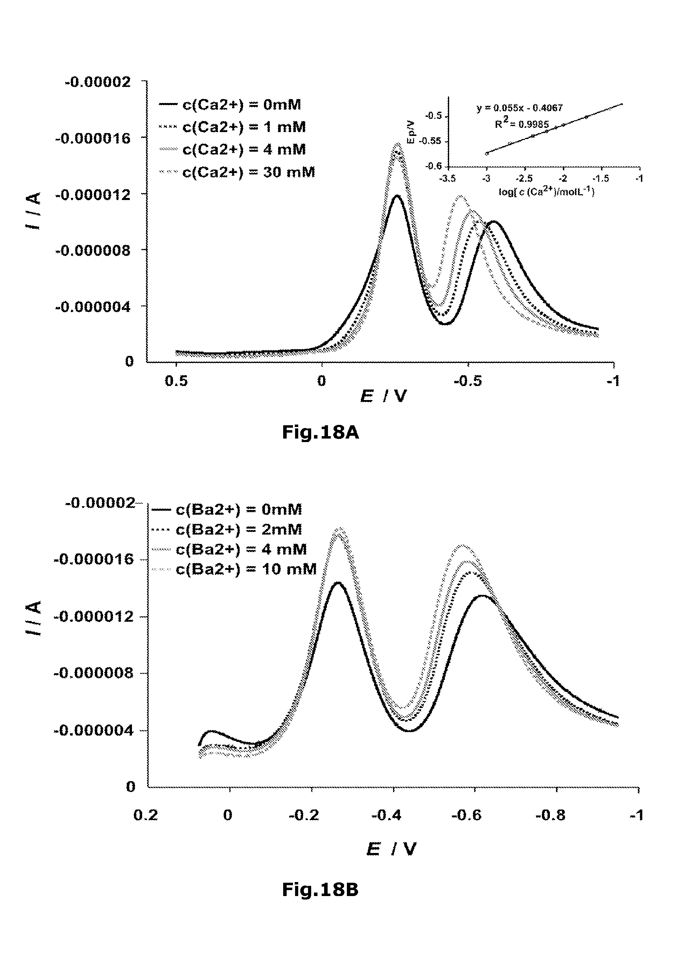

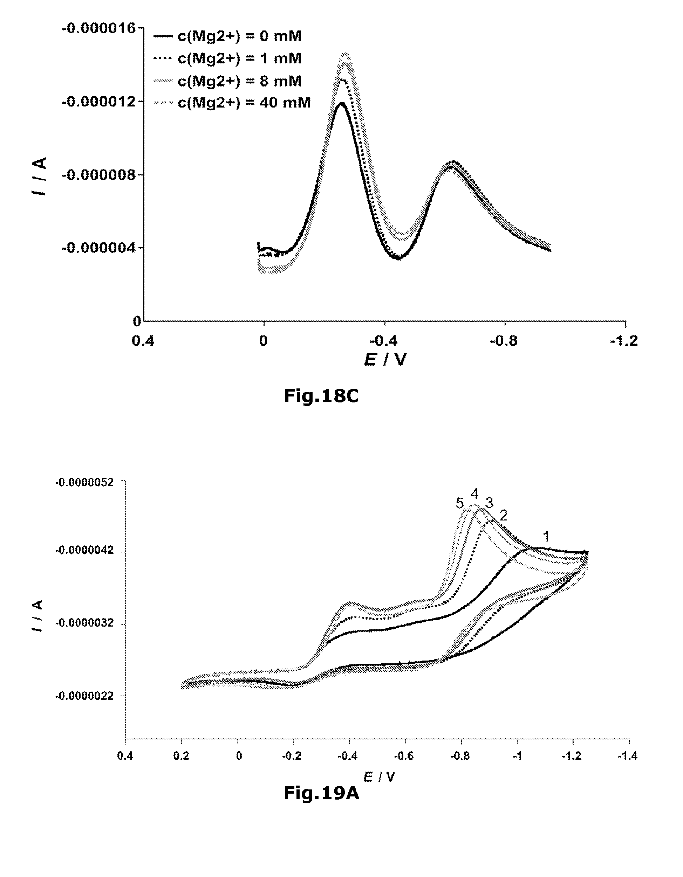

Fig. 18: Square-wave voltammograms of the synthesized O-demethylated benzoquinone (c = 1 mmol/l) showing its sensitivity (the peak at more negative potentials) to several earth-alkaline cations. A: Ca²⁺; B, Ba²⁺; and C: Mg²⁺. The pH of the solutions was 7.40, scan rate = 30 mV/s. The peak at more positive potentials originates from the native (yellow form) of 2,6-dimethoxy 1,4-benzoquinone, and it is completely insensitive to the concentrations of the earth-alkaline cations.
Fig. 19 A: Cyclic voltammograms of 1 mmol/l of synthesized O-demethylated form of 2,5-dimethoxy 1,4-benzoquinone showing its sensitivity to Ca²⁺ ions (the peak at more negative potentials). c(Ca²⁺)/mmol●l⁻¹ = 0(1), 1(2) 3(3) 6(4) and 10(5); scan rate = 30 mV/s. pH = 7.40; B: Square-wave voltammograms of O-demethylated Coenzyme Q0 showing its sensitivity to Ca²⁺ ions. Square-wave amplitude was 50 mV, Square-wave frequency was 10 Hz; pH = 7.40; The peaks at more positive potentials originate from the native (yellow forms) of 2,5-dimethoxy 1,4-benzoquinone (A) and Coenzyme Q0 (B), and they are completely insensitive to various concentrations of earth-alkaline cations.

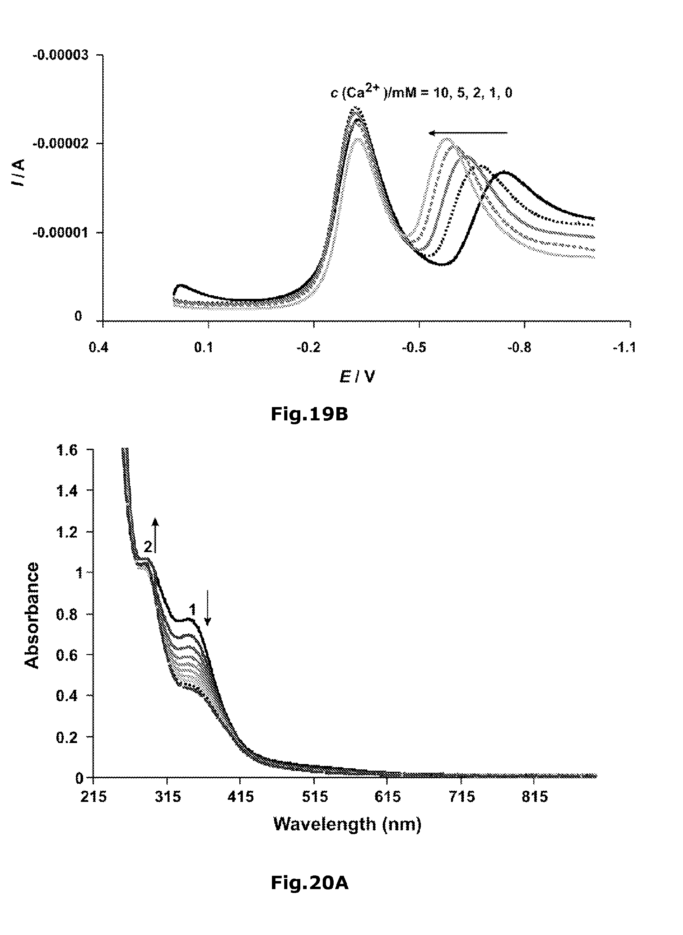

Fig. 20 A: UV-VIS spectrum of 50 pmol/l reduced form of synthesized O-demethylated benzoquinone from Example 4 showing its reoxidation with oxygen within a period of 24 h in a pH of 7.00. B: UV-VIS spectrum of 50 µmol/l reduced native form of 2,6-dimethoxy 1,4-benzoquinone from Example 1 showing its reoxidation with oxygen within a period of 24 h in pH of 7.00.

### Detailed Description of the Invention

In the compounds of aspects (1), (2) and (3) of the invention also include tautomeric forms of the structure shown in formula (I).

Moreover, it is preferred that the remaining residues of R¹-R⁴ and R¹ and R² are independently selected from H, C₁-₆alkyl, C₂₋₆alkenyl, and C₁-₆alkoxy. Particularly preferred are H, C₁-₄alkyl, C₂₋₄alkenyl, and methoxy.

The C₁₋₆alkyl includes straight, branched and cyclic alkyl residues having one to six carbon atoms, including but not limited to methyl, ethyl, n-propyl, isopropyl, cyclopropyl, n-butyl, sec-butyl, tert-butyl, cyclobutyl, n-pentyl, sec-pentyl, neopentyl, cyclopentyl, n-hexyl, sec-hexyl and cyclohexyl.

The C₂-₆alkenyl includes unsaturated straight, branched and cyclic alkenyl residues having two to six carbon atoms, e.g. unsaturated forms of the C₁-₆alkyls mentioned hereinbefore, including vinyl, allyl, isoprenyl, and so on.

The C₁-₆alkoxy includes straight, branched and cyclic alkoxy residues having one to six carbon atoms, including those mentioned with respect to the C₁-₆alkyl hereinbefore.

Alternatively, two of the residues R¹-R⁴, if adjacent, or R¹ and R², if adjacent, together with the two carbon atoms of the benzoqinone ring, may form a carbocyclic or heterocyclic ring which may be saturated, unsaturated or aromatic. The carbocyclic or heterocyclic ring may be a 5-7 membered ring, with N, O and/or S as heteroatoms, may be saturated, unsaturated or aromatic, and includes a cyclopentenyl, cyclohexenyl, phenyl and pyridyl ring.

The C₁₋₆alkyl, C₂₋₆alkenyl, C₁₋₆alkoxy and the carbocyclic or heterocyclic ring may each carry one to three substituents, which are independently selected from C₁-₄alkyl, C₁₋₄alkoxy, hydroxy, carboxy, oxy and amino, and, if the cabocyclic and heterocyclic ring is aromatic, may further be selected from halogen and nitro. Particularly preferred compounds of formula (I) are 2,3-dioxy-6-methoxy-para benzoquinone, 2,3-dioxy-6-methyl parabenzoquinone (O-demethylated Coenzyme Q0) and 2,3-dioxy-5-isoprenyl-6-methyl-parabenzoquinone (O-demethylated Coenzyme Q1) and salts thereof, the structures thereof being shown in Table 1.

**Table 1: Structures and IUPAC names of the parent quinones, and of the O-demethylated products.**

| **Structure of parent compound** | **Name** | **Structure of product compound** | **Name** |
|---|---|---|---|
| 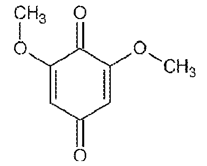 | 2,6-dimethoxy-para-benzoquinone | 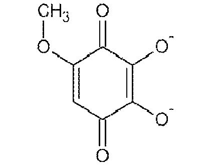 | 2,3-dioxy-6-methoxy -para-benzoquinone |
| 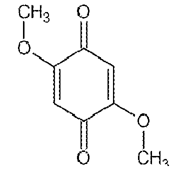 | 2.5-dimethoxy-para-benzoquinone | 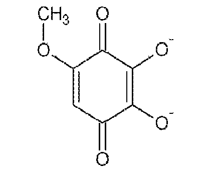 | 2.3-dioxy-6-methoxy-para-benzoquinone |
| 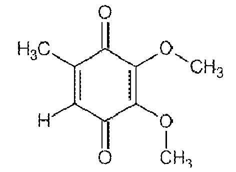 | Coenzyme Q0 | 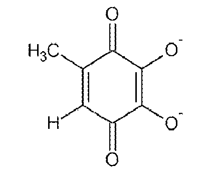 | 2.3-dioxy-6-methyl-para-benzoquinone |
| 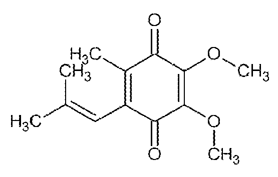 | Coenzyme Q1 | 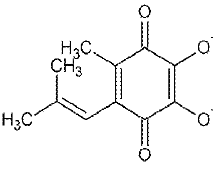 | 2,3-dioxy-5-isoprenyl-6-methyl-para-benzoquinone |

In another preferred embodiment of aspects (1), (2) and (3) the cation forming the salt is selected from earth alkaline metal cations including Mg²⁺, Ca²⁺, Ba²⁺, and Sr²⁺ ions.

The above mentioned O-demethylated compounds in Table 1 have the following stability constant (*K*_{stab}.) with earth-alkaline metals:
K_{stab}. = 1.95 x 10⁹ mol⁻¹ I³ for Ca²⁺; *K*_{stab}. = 6.95 x 10⁷ mol⁻¹ I³ for Ba²⁺; K_{stab}. = 1.45 x 10⁶ mol⁻¹ I³ for Sr²⁺; and K_{stab}. = 4.05 x 10⁴ mol⁻¹ I³ for Mg²⁺ ions (for 2,3-dioxy-6-methoxy-para benzoquinone); and
K_{stab} = 1.05 x 10⁸ mol⁻¹ I³ for Ca²⁺; K_{stab} = 4.50 x 10⁶ mol⁻¹ I³ for Ba²⁺; K_{stab} = 2.70 x 10⁶ mol⁻¹ I³ for Sr²⁺; and K_{stab} = 2.00 x 10⁴ mol⁻¹ I³ for Mg²⁺ ions (for 2,3-dioxy-6-methyl parabenzoquinone).

It is furthermore preferred that the compounds of the invention are highly soluble in water, preferably more than 0.05 mol/l, but highly insoluble in lipophilic organic solvents including 1,2-dichlorethan, nitrobenzene, and nitrophenyl octyl ether and hardly soluble in alcohols.

It is furthermore preferred that the compounds of the invention show long-lasting stability, when retitrated to neutral within 12 h after its production.

It is furthermore preferred that the compounds of the invention form an at least - 300 mV stronger antioxidant than the corresponding parent compounds (i.e. the reduced forms of 2,6-dimethoxy benzoquinone, 2,5-dimethoxy benzoquinone, 2,3-dimethoxy-6-methyl benzoquinone, and Coenzyme Q1) in neutral media.

It is furthermore preferred that the compounds of the invention are insensitive to pH in the regions 4.5 < pH < 13.0.

It is furthermore preferred that the compounds of the invention are unable to form complexes with mono-charged alkaline cations Li⁺, Na⁺, K⁺, Rb⁺, Cs⁺ and NH₄⁺.

It is furthermore preferred that the compounds of the invention are selective ligands for Ca²⁺and Mg²⁺ ions in neutral media.

In the method of aspects (4) and (5) of the invention the variables of the (dimethoxy) benzoquinone derivatives of the formula (I) 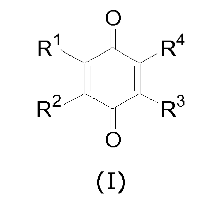 have the following meaning: two out of R¹ to R⁴ are as defined in connection with aspect (1) above, and the other two are independently selected from H and alkoxy, including C₁-₄alkoxy. The C₁-₄alkoxy includes methoxy, ethoxy, etc. as set forth above.

Particularly preferred compounds of formula (I) for preparing the particularly preferred compounds of aspects (1) to (3) include 2,6-dimethoxy benzoquinone and 2,5-dimethoxy benzoquinone (for preparing 2,3-dioxy-6-methoxy-para benzoquinone), 2,3-dimethoxy-5-methyl benzoquinone (Coenzyme Q0) and 2,3-dimethoxy-5-isoprenyl-6-methyl-para-benzoquinone (Coenzyme Q1), the structures of which are shown in Table 1.

The reaction is performed by subjecting the compound of formula (I) to a reaction with a concentrated aqueous alkaline solution, including an aqueous NaOH, KOH or LiOH solution. It is preferred that such reaction is performed at pH 11 to 13, at a temperature of -15 to 30 °C, preferably at 20 to 25 °C, for 20 min to 24 h, preferably 30 min to 12 h, most preferably 30 minutes to 8 h.

The method may further comprise retitration of the O-demethylated product to neutral, preferably at least 20 minutes after the initiation of the O-demethylation reaction.

The composition, reduction agent or stabilizer comprising the compounds of the invention of aspects (1), (2) and (3) of the invention may further contain NaCl, KCl and alcochol.

According to aspect (7) of the invention the compound of the invention is used for preparing medicaments having antioxidant properties. Such medicament is applicable for treating age and free radical induced diseases.

According to aspect (8) of the invention the compounds of the invention can be used as antioxidants, anti-ageing agents, in organic synthesis, for metal complexations, metal separations, potentiometric titrations, interfacial catalysis, and ion-transfer reactions across biological membranes. Here the compounds of the invention may be applied together with compounds known for that purpose.

Thus the invention further includes a method for treating age and free radical induced diseases in a patient, said method comprises administering the patient a suitable amount of the compound of the invention of aspects (1), (2) and (3) of the invention.

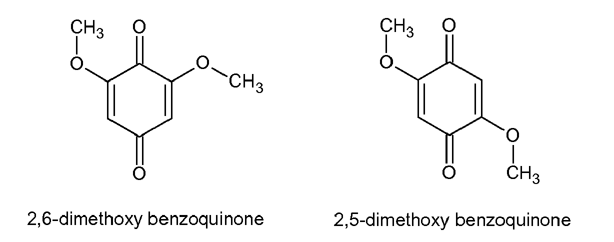

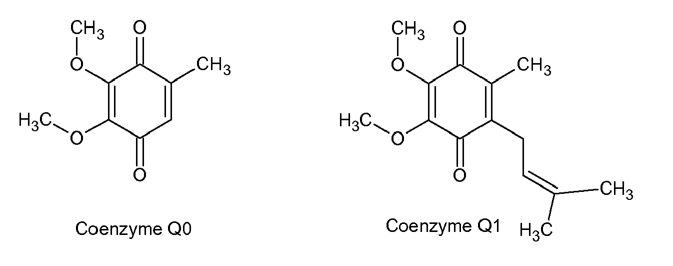

### Scheme 1: Structures of the preferred starting compounds.

In the following, the behavior of quinones in alkaline media leading to generation of O-demethylated quinone forms is described in more detail as well as their distinct physico-chemical properties. Because the behavior of all investigated compounds is relatively similar, we describe in details only the results for Coenzyme Q1, 2,6-dimethoxy benzoquinone and Coenzyme Q0 shown in scheme 1 above. It is however to be understood, that the invention is not limited to said compounds.

Coenzyme Q1, Chemical and electrochemical properties of CoQ1 initially dissolved in acidic and neutral aqueous media: Coenzyme Q1 (CoQ1, also named as Ubiquinone 5) is an analog of CoQ10 and contains only one instead of ten isoprenoid groups in its side chain. The native CoQ1 is rather lipophilic and poorly water-soluble substance (its solubility in water is lower than 100 pmol/l, giving yellowish color of the solutions (Rich, P.R., Harper, R. FEBS Lett. 269(1):p.139-44 (1990)). The voltammetric behavior of CoQ1 dissolved directly in water solutions having pH from 1 to 9 is similar to that of other quinones (Morron, R., Biochemistry of Quinones, New York-London: Academic Press Inc. (1965); Swenton J., Chemistry of Quinones, New York: John Wiley (1988); Quan, M. et al., J. Am. Chem. Soc. 129(42:p.12847-56 (2007); Chambers, J., The Chemistry of Quinonoid Compounds, John Wiley & Sons, p. 737-791 (1974); rich, P.R. Biochimica et Biophysica Acta (BBA)-Bioenergetics 637(1):p.28-33 (1981)).
Specifically, depending on pH, their cyclo-voltammetric response consists of one or two pairs of quasireversible peaks, whose mid-peak potentials shift toward more positive potentials by increasing the H⁺ concentration. In aqueous solution at around neutral pH's, the only stable forms of most quinone systems are the fully protonated quinol QH₂ (hydroquinone), and the neutral quinone, Q. The overall redox transition involves the transfer of two protons and two electrons. Mechanistically, however, the change commonly occur in successive one-electron and one-proton steps via the semiquinone intermediates, and this leads to a diverse range of possible reaction routes and intermediates, depending on the order in which the protons and electrons are transferred. The dominant route followed can easily be modulated by a change of solution conditions (pH) and quinone concentration (Quan, M. et al., J. Am. Chem. Soc. 129(42:p.12847-56 (2007)). The cyclic voltammograms (CV) of CoQ1 directly dissolved in aqueous buffers having pH of around 7 consist of a single voltammetric process, with mid-peak potential of Emid = -0.36 V vs. Ag/AgCI. In such scenario, the redox process of native "yellow" form of CoQ1 was sensitive only to proton concentration, but not at all to the concentrations of the alkaline or earth-alkaline metal cations.

Chemical and electrochemical features of CoQ1 initially dissolved in strong alkaline media: Quite different chemical and electrochemical behavior was observed for CoQ1, when it was initially dissolved in strong alkaline media. The dissolution of CoQ1 in 1 mol/l NaOH was followed by a rapid conversion of the color solution from yellowish via rosé-wine to intensive violet. We supposed that these changes were due to a chemical reaction between CoQ1 and the nucleophilic OH- ions (present in large concentration in the alkaline solution). To verify this, we used cyclic and square-wave voltammetry, UV-VIS, EPR and NMR techniques.

The cyclic voltammograms of CoQ1 recorded at different time points following its dissolution in 1 mol/l NaOH consist of two reversible redox processes, whose intensities are significantly affected by time. While the height of the first pair of peaks at more positive potentials decreases with time, the intensity of the second pair of peaks at more negative potentials increases concomitantly. After period of 8 h in 1 mol/l NaOH, the first voltammetric redox process of CoQ1 diminished significantly, while the second redox process at more negative potentials was significantly increased. These experiments implicated that after period of about 8 h, the reaction between CoQ1 and NaOH was significantly shifted toward the created product, and the final product of the reaction between NaOH and Coenzyme Q1 was predominant fraction in the solution. Once being formed in the strong alkaline solutions, the product of the reaction between CoQ1 and OH- is very stable, if the solution is re-titrated to neutral and acidic pH's up to 12 h after the reaction was initiated.

At this stage, it is also worth to refer on the hydrophilic properties of the product formed by reaction of CoQ1 and NaOH. It is well known that native CoQ1 is a nicely soluble in various organic solvents, but poorly in water. The solubility of CoQ1 in water is lower than 100 µmol/l. However, its solubility in strong alkaline media increases dramatically, even over 0.01 mol/l This is additional indicator that CoQ1 reacts with NaOH, giving as a product a nicely water-soluble compound. The good water solubility of the product of reaction between CoQ1 and NaOH should be inevitably linked with highly increased polarity of the synthesized product. Additional evidences for the changes in the structure of CoQ1 when dissolved in strong alkaline solutions have been provided by UV-VIS spectrophotometrical experiments. The spectrophotometric measurements of CoQ1 dissolved in 1 mol/l NaOH (performed in kinetic mode) revealed evident changes in the absorption bands with the time, signifying formation of a new compound. The initial UV-VIS spectrum of CoQ1 dissolved in 1 mol/l NaOH (after 5 min) consist of 2 absorption bands located at around 280 nm and around 325 nm, alongside to the band located at about 220 nm that is due to the absorption of the aromatic ring. The band at 280 nm was due to the absorption of native form of CoQ1, while the band at 325 nm was due to the absorption of the product created via the reaction of CoQ1 and NaOH. While the intensity of the first absorption band at 280 nm decreases with the time, the intensity of the second band at 325 continuously augments for same extent. After period of 4 h the chemical equilibrium is significantly shifted toward the product of the chemical reaction, yielding mainly a broad intensive absorption peak at 325 nm: These experiments were consistent to the electrochemical ones, and they are good qualitative indicators for the existence of chemical reaction between CoQ1 and NaOH in strongly alkaline media.

Revealing the structure of the reaction product of 01 and NaOH by EPR and NMR experiments: The ability of quinones to accept two electrons (and two protons) in consecutive steps allows use of electron paramagnetic resonance (EPR) to monitor the one electron reduced radical state of the molecule. It was shown recently, that in alkaline conditions benzoquinones undergo reduction induced by hydroxide ions. In particular, the formation of dianions with reducing properties was postulated (Pedersen, J.A., Spectrochim. Acta A Mol. Biomol. Spectrosc. 58(6):p.1257-70 (2002)). EPR experiments were performed for neutral and basic solutions Q1. In neutral aqueous solution, no spontaneous radical production was found, but when reduced with sodium borhydrid (NaBH₄) a specific EPR signal of 9 lines was observed. This signal is caused by a methyl group, and one CH₂ group (Fig. 7). Because of the intrinsic asymmetry of Q1 (as compared to e.g. 2,6-dimethoxy benzoquinone), the CH₃-protons of the methoxy-group have very small unresolvable couplings. Under strongly basic conditions, an identical line pattern was observed. However, the radical signal showed pronounced time dependence and appeared after about 5 min building up intensity (i.e. radical concentration) for about two hours. It was stable for several hours. According to the literature on the radical chemistry of various benzoquinones (Pedersen, J.A., Spectrochim. Acta A Mol. Biomol. Spectrosc. 58(6):p.1257-70 (2002)) tetra-substituted compounds should not form radicals in basic media, because the addition of OH- as a crucial step to produce a di-anion cannot occur. This step is thought to be a prerequisite for the electron transfer from the di-anion to the parent compound which constitutes the EPR radical signal. Because all four ring positions in Coenzyme Q1 are occupied with methoxy-, methyl- or alkyl-groups, the formation of radicals in basic conditions is surprising and needs an alternative explanation. The possibility of a cleavage of the methyl- or isoprenoid group was tested with 2-methyl benzoquinone in strong alkaline solutions. As expected a radical EPR signal was observed, which always contained a methyl-group contribution. This indicates that such ring substituents cannot be cleaved under the applied conditions. The remaining possibility to form di-anions is the cleavage of the methyl-groups of the methoxy substituents by OH- associated with the formation of methanol. This assumption was verified by NMR experiments, for which samples of Coenzyme Q1 were reacted in alkaline solution for defined times and then re-titrated to neutral pH to stop the reaction. It was found that a loss of the NMR signal of the methoxy-protons was directly correlated to the appearing signal of methanol (Fig. 8, 9). EPR and NMR experiments thus give clear evidence that in alkaline solution the di-anion of Q1 is formed by demethylation of the methoxy groups, which is capable to reduce the parent compound to a radical species. The reaction to the double negatively charged di-anion form is represented in the scheme 2:

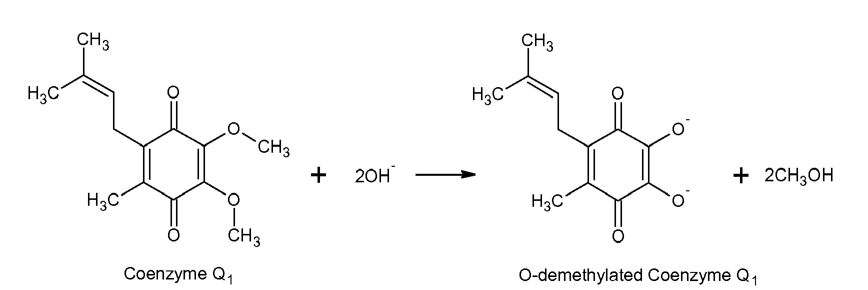

### Scheme 2: Reaction of scission of two methoxy groups in CoQ1 structure by means of OH- in strong alkaline media.

The two charged groups in the O-demethylated structure are actually responsible for the highly increased solubility of Coenzyme Q1. This new form of Coenzyme Q1 can be considered as the double dissociated 2,3-dihydroxyl-5-methyl-6-isoprenyl benzoquinone. The presence of two negatively charged "O-"groups in the '0-demethylated" structure of Coenzyme Q1 results in completely new properties of this compound in respect to its parent compound.

It noted that prolonged reaction times (approximately 18 h) in alkaline media lead to very complex radical-radical reactions, giving systems with very complex structures as products. In order to obtain the desired product of scheme 2, a retitration of the alkaline solution is recommended for up to maximally 12 h after initiation of the reaction.

Cyclo-voltam metric behavior of CoQ1 initially dissolved in 1 mol/l NaOH; Proton insensitivity and calcium sensitivity: In experiments performed with CoQ1 initially dissolved for certain time (15 min to 8 h) in strong alkaline media, a remarkable pH-dependent behavior of the cyclic voltammograms has been observed. The cyclic voltammograms of CoQ1 initially dissolved in 1 mol/l NaOH for ~ 60 min, and retitrated afterwards to different lower pH's showed quite interesting features. In solutions having pH from 3.0 to 12.0, two redox processes at the cyclic voltammograms exist. The first redox process (at more positive potentials) originates from the native (parent) form of CoQ1, and its mid-peak potential is mainly proton sensitive. Interestingly, the mid-peak potential of the second electrode process (at more negative potentials) was insensitive to proton concentration in pH regions from 4.5 to 12. This is a quite unusual finding, since it is well known that the redox chemistry of most quinones is sensitive to proton concentration in mentioned pH's region. The origin of the redox process at very negative potentials was the reduction-oxidation of the negatively charged O-demethylated CoQ1 form. The highly negative potential needed to reduce this product is, indeed, consistent with the huge electronic density present in its structure. The insensitivity of the second redox process to H⁺ concentration implies that the contributors in this second redox process are strongly stabilized via strong hydrogen bonds with the solvent molecules. As recently confirmed by [Quan, M. et al., J. Am. Chem. Soc. 129(42:p.12847-56 (2007), and (Manojkumar, T.K. et al., The Journal of Chemical Physics 118(19):p.8681-8686 (2003)], the hydrogen bonding of quinone-anionic forms to water molecules undoubtedly plays important role in the quinone's electrochemistry.

Although it showed no sensitivity to proton concentration, we found that the reduced form of the O-demethylated CoQ1 possesses intrinsic properties to complexate earth-alkaline cations (Ca²⁺, Sr²⁺ and Ba²⁺, and to smaller extent Mg²⁺) in neutral media, but not at all the monocharged alkaline cations Na⁺, K⁺, Cs⁺, and Li⁺. The voltammetric process of O-demethylated CoQ1 (the process happening at more negative potentials) showed quite obvious sensitivity to the concentration of Ca²⁺ cations. The mid-peak potentials (Δ*E*_{p,mia}) of cyclic voltammograms representing the redox process of O-demethylated CoQ1 were linear function of the logarithm of the Ca²⁺ concentration, with slope of that dependence having a thermodynamic value of 61 mV/log[c(Ca²⁺)]. This finding implies that a stochiometric complex of type 1:2 between the electrochemically reduced O-demethylated CoQ1 and Ca²⁺ ions is formed. The intercept of the dependence Δ*E*_{p,mia} *VS.* log[c(Ca²⁺)] hides the value of the stability constant of the complex, which was estimated to be 6.60 x 10⁶ mol⁻¹ I³. Accordingly, the reaction of reduction and complex-formation of the O-demethylated CoQ1 and Ca²⁺ ions can be presented with following reaction scheme 3:

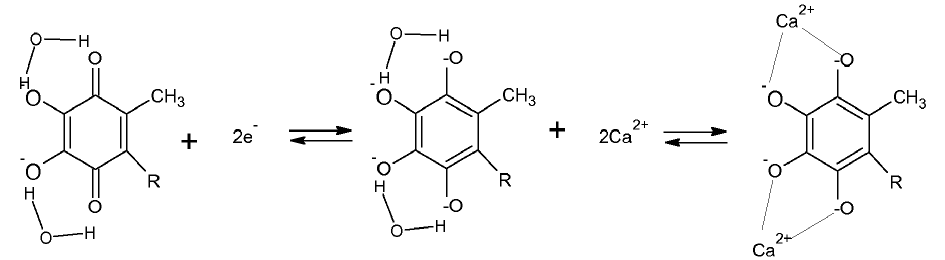

Scheme 3: Reduction of the O-demethylatated form of CoQ1 and its complexation process with Ca²⁺ ions.

Qualitatively similar properties as those described for Coenzyme Q1 we also observed for Coenzyme Q0.

2,6-dimethoxy benzoquinones; Experiments with dimethoxy benzoquinones initially dissolved in neutral media: In majority of the reported studies, the investigated quinone-like compounds are initially dissolved in water (or in mild acidic solutions) (Bartlet, P.N., Bioelectrochemistry; fundamentals, experimental techniques, and applications: Wiley (2008); Chambers, J. The Chemistry of Quinoid Compounds, Wiley: New York, p. 537-717 (1988)). Under these conditions, the color of the aqueous solutions of all dimethoxy benzoquinones is intensively yellow. Aliquots taken from stock solutions are placed in buffered electrolyte solutions. Subsequently, electrochemical experiments are conducted by using cyclic or square-wave voltammetry. The cyclic voltammograms of 2,6-dimethoxy para-benzoquinone recorded in buffer solutions with different pH values (pH was changed in the region from 1.30 to 9.00) show single quasireversible redox process. The mid-peak potentials of cyclic voltammograms are shifting by +60 mV per pH unit. This behavior corresponds to the well-known redox chemistry of quinones in aqueous media, in which two electrons and two protons are mainly involved (see reaction scheme 4).

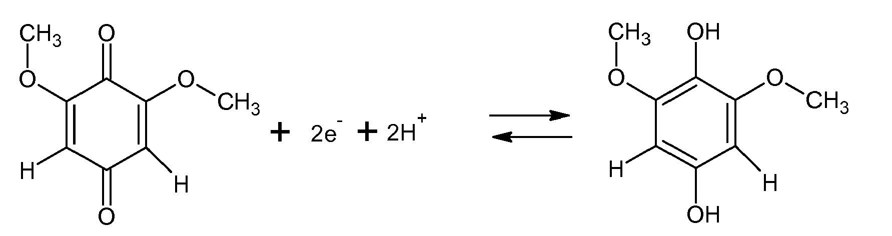

### Scheme 4: Redox chemistry of 2,6-dimethoxy benzoquinone in acidic and neutral media.

In this case, the redox process of 2,6-dimethoxy benzoquinone was found to be sensitive only to protons, but not at all to alkaline or earth-alkaline metal ions.

Experiments with dimethoxy benzoquinones initially dissolved in strong alkaline solutions; Getting insights into the chemical reaction between 2,6-dimethoxy 1,4-benzoquinone and NaOH by cyclic voltammetry: For 2,6-dimethoxy 1,4-benzoquinone dissolved in strongly alkaline media (1 mol/l NaOH), a very rapid conversion of the solution color from yellow via rosé wine to a dark-red was observed. Such a drastic change in color indicates that the native compound undergoes significant alterations in its electronic structure. The cyclic voltammograms of 2,6-dimethoxy benzoquinone recorded shortly after dissolving it in alkaline solution of 1 mol/l NaOH are featuring two reversible redox processes (i.e. two reversible peaks) separated by about 250 mV, whose intensities are significantly affected by time. While the height of the first peak (at more positive potentials) decreased, the intensity of the new second peak at more negative potentials increased concomitantly. After period of about 1 day in 1 mol/l NaOH solution, the first voltammetric peak vanishes almost completely, while mainly the second voltammetric process at more negative potentials is persisting. These experiments implied that after period of 24 h, the reaction between 2,6-dimethoxy benzoquinone and NaOH is significantly shifted to the product, and the product of the chemical reaction is main fraction in the solution. The rate of the chemical reaction was significantly smaller when the pH of the solutions was changed from 13 to 11. However, once being formed in the strongly alkaline solutions, the product of the reaction between 2,6-dimethoxy benzoquinone and OH- was very stable when re-titrated to neutral or slight acidic pH's after period of 30 min to 12 h from the initiation of the reaction. This can be recognized from the color of those solutions, as well as from the features of the voltammetric curves.

Getting insights into the chemical reaction between 2,6-dimethoxy 1,4-benzoquinone and NaOH by UV-VIS experiments: Additional evidences for the reaction of 2,6-dimethoxy benzoquinone induced in strongly alkaline solutions are obtained by UV-VIS spectrophotometrical experiments. Measurements in kinetic mode revealed clear changes in the absorption spectra, and formation of a new compound. The UV-VIS spectrum of 2,6-dimethoxy 1,4-benzoquinone in neutral media consists of one band located at 250 nm. Shortly after dissolving of 2,6-dimethoxy benzoquinone in 1 mol/l NaOH, one more absorption band was observed in the UV-VIS spectrum, positioned at about 300 nm. The intensity of the band at shorter wavelengths decreases, while the intensity of the band at higher wavelengths increases. After period of 24 h, the only existing band is that at wavelengths of 300 nm. This means, that the native 2,6-dimethoxy benzoquinone (the absorption band at 250 nm) is almost completely converted to a new product (the absorption band at 300 nm) via the reaction with OH- ions. The results of the UV-VIS experiments are complementary with the aforementioned electrochemical results.

Insights into the hydrophilic properties of the synthesized product of the reaction between 2,6-dimethoxy benzoquinone and NaOH: It is well known that the methoxy benzoquinone derivatives are amphiphilic compounds, soluble to some extent in water, but easily soluble in various organic solvents. The solubility of 2,6-dimethoxy benzoquinone in water or in mild acidic and basic media is moderate, i.e. up to 1.5 mmol/l. However, its solubility in strongly alkaline media increases dramatically to more than 50 mmol/l. This is another very strong indicator that 2,6-dimethoxy benzoquinone reacts with NaOH, giving as product a nicely water-soluble compound. The good water solubility of the product must be inevitably linked with the highly increased polarity of the synthesized product. A set of simple extraction (i.e. shake-flask) experiments were performed for (1) a pure 1,2-dichlorethan (a rather non-polar, water immiscible organic solvent) and an aqueous solution of 2,6-dimethoxy benzoquinone dissolved directly in pH of 7.0; and for (2) pure 1,2-dichlorethan and alkaline aqueous solution (1 mol/l NaOH) of 2,6-dimethoxy benzoquinone, to monitor the partitioning behavior. In case (1) the neutral 2,6-dimethoxy benzoquinone (yellow form) distributes clearly between the water and the organic solvent, while in case (2) there is no partition to the organic solvent at all. It is concluded that the reaction product in alkaline media must be a highly polar (charged) stable compound. The highly polar structure of the synthesized product is the origin for its extensive solubility in water, and its complete insolubility in non-polar organic solvents.

Revealing the structure of the product of the reaction between 2,6-dimethoxy 1,4-benzoquinone and NaOH: From an EPR spectroscopic point of view 2,6-dimethoxy benzoquinone is different from CoQ0 or CoQ1 mainly with respect to the intrinsic molecular symmetry (see scheme 1). According to literature (Pedersen, J.A., Spectrochim. Acta A Mol. Biomol. Spectrosc. 58(6):p.1257-70 (2002)) it is used as a benzoquinone model to study the reduction induced in strongly alkaline solutions, and, in particular, to characterize the postulated di-anion forms with reducing properties. EPR experiments were performed for neutral and basic solutions of 2,6-dimethoxy benzoquinone. In neutral media no spontaneous radical formation was observed, but the molecule could be reduced by half-equimolar amounts of sodium borhydrid. The obtained characteristic 11-line spectrum is consistent with the presence of the two methyl-groups of the methoxy substituents (six methyl-protons: 0.805 G) and two equivalent ring protons (two H: 1.465 G) (Fig. 7). It is noted that in contrast to CoQ0 or CoQ1 the methyl protons of the methoxy-groups now have sizeable coupling values and can be easily resolved for this high symmetry molecule. In the presence of OH- at pH 13 after a lag time of about 45 min a new signal was observed with a typical pattern composed of a methyl-group (0.69 G), one larger (2.51 G) and one small (0.206 G) proton contribution. In deuterated basic solvent (NaOD, D₂O) the small proton coupling was lost indicating that a ring proton is substituted by an OD-group or by the hydroxyl anion in case of NaOH, H₂O solvent. The initial signal is growing rapidly and persists for more than three hours. The results show clearly that the methyl-group of one methoxy moiety is cleaved leaving negatively charged oxygen. In addition, the attached OH-group is also forming a negatively charged group on the ring in basic conditions (see Reaction scheme 5 below). The ongoing reaction in strongly basic medium can be quenched by a retitration to neutral pH. Hence, by choosing the appropriate time the degree of demethylation and OH- addition can be determined.

The product of the O-demethylation should be methanol. Its production depending on the time of retitration was studied by NMR. Plotting the ratio methanol/methoxy of signal integral gives a similar curve as for CoQ0 (Figs. 8 and 9). It clearly demonstrates that in the time course methanol is produced by cleaving the methyl-group off the metoxy-substituents.

The presence of nucleophilic OH- ions in high concentration provokes significant changes in the structures of the studied methoxy benzoquinones. Specifically, the OH- ions can cleave the etheric bonds in the dimethoxy benzoquinones, while creating very polar (negatively charged) quinones structures that are highly stabilized via the hydrogen bonding with the solvent molecules. The simplified sequence of the alkaline synthesis of O-demethylated benzoquinone forms can be presented by following reaction scheme 5:

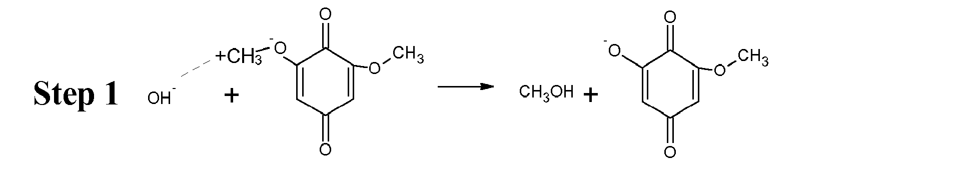

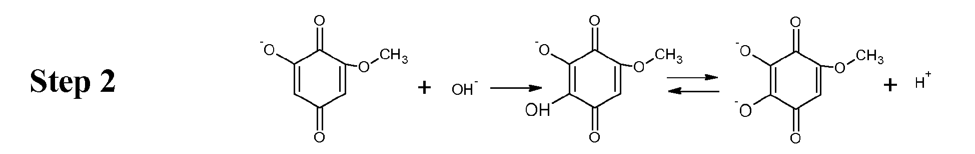

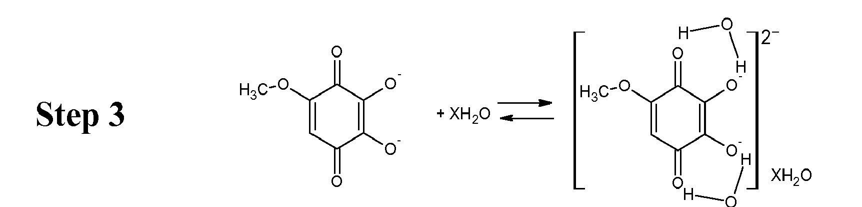

### Scheme 5: Possible chemical steps involved in reaction of dimethoxy benzoquinones and NaOH.

In the first step, the highly nucleophilic OH- anions will attack the positive part of one of the polar methoxy groups in the methoxybenzoquinone structure, contributing in that way to the scission of one methoxy group (Step 1). The second step comprises substitution of one OH group at the free position in the aromatic ring of the methoxy benzoquinones, creating in that way a hydroxy-substituted benzoquinone. Due to the acidity of the OH group substituted in the aromatic ring, dissociation (deprotonation) of this group in alkaline media will take place, giving as a product a double negatively charged benzoquinone anion (Step 2). Indeed, the sequence of steps 1 and 2 might also be reversed. The third step is stabilization of the negatively charged O-demethylated 1,4-benzoquinone dianionic structure via the hydrogen bonding formed with the highly polar water molecules (Step 3).

Cyclo-voltammetric experiments showing pH insensitivity of the synthesized compound: The O-demethylated benzoquinone products, synthesized via the reaction of the dimethoxy 1,4-benzoquinones with NaOH, show a quite different pH-dependent cyclo-voltammetric behavior than the native (yellow form) of dimethoxy 1,4-benzoquinone. In the following the results from experiments are discussed, in which a 1 mmol/l of 2,6-dimethoxy 1,4-benzoquinone was initially let to react with 1 mol/l NaOH solution for 60 min. Subsequently, the pH of the solution was adjusted to the desired values by titration with 1 mol/l nitric acid (HN0₃), and cyclic voltammograms were recorded. In aqueous solutions having pH > 4.5, we observed two quasireversible redox processes. The first redox process (appears at more positive potentials) originates from the native (yellow form) of 2,6-dimethoxy benzoquinone, and its mid-peak potential was a proton sensitive item in large region of pH's (from pH 1.0 to pH of 10.0). The behavior of this redox process is known from the previous similar studies on quinone-like systems [Quan, M. et al., J. Am. Chem. Soc. 129(42:p.12847-56 (2007)], and it is in accordance with the reported quionone's properties [Morron, R., Biochemistry of Quinones, New York-London: Academic Press Inc. (1965); Rahman, A., Bioactive Natural Products, ed. Rahman:Elvesier (2002); Swenton J., Chemistry of Quinones, New York: John Wiley (1988)]. However, the mid-peak potential of the second voltammetric process (it appears at more negative potentials than the first redox process i.e. at about -0.7 V) was almost completely insensitive to proton concentration over a wide range of pH's (i.e. from pH = 13 to pH = 4.5). The origin of this second redox process is the reduction-oxidation of the synthesized benzoquinone product (O-demethylated red form), created via the chemical reaction between 2,6-dimethoxy BzQ and OH- in strongly alkaline solutions. The highly negative potential of about -0.70 V needed to reduce the synthesized O-demethylated benzoquinone compound is, indeed, consistent with the huge electronic density present in its structure. Moreover, the insensitivity of the second redox process to the proton concentration implies that the electrochemical products of negatively charged benzoquinone derivative must be stabilized via strong hydrogen bonds with the water molecules. As recently confirmed (Quan, M. et al., J. Am. Chem. Soc. 129(42:p.12847-56 (2007); Manojkumar, T.K., The Journal of Chemical Physics 118(19):p.8681-8686 (2003)) the hydrogen bonding of quinone anionic forms to water molecules undoubtedly plays important role in the quinones electrochemistry. Based on the three lone electron pairs placed on each oxygen, and the two additional free electrons at the demethylated and dissociated oxygens, one can estimate that at least 12 to 14 strong-to-moderate hydrogen bonds are possible. This effect certainly brings stabilization of the polar O-demethylated benzoquinone product, while also contributing to its high water solubility. Next to the high potential for hydrogen bonding, the charge delocalization should also play a significant role into the features of the O-demethylated forms of studied benzoquinones. While both charged oxygen atoms in the O-demethylated benzoquinone structure bear a net charge of "2-", that charge is not exclusively localized at those specific oxygen atoms; rather it is dispersed over all oxygens in the conjugated O-demethylated aromatic structure. In such situation, several resonance forms for its structure can be considered. Such charge delocalization is a common feature of conjugated resonance structures of many polar quinone-like aromatic compounds, and it is additional possible cause for observed proton insensitivity of the O-demethylated benzoquinones.

The synthesized O-demethylated 1,4-benzoquinones compounds are strong and selective ligands for the earth-alkaline cations: Although it showed no sensitivity to proton concentration, we found that the reduced form of the synthesized O-demethylated 1,4-benzoquinone possesses intrinsic properties to complexate earth-alkaline cations (Ca²⁺, Ba²⁺, Sr²⁺ and to much smaller extent Mg²⁺) in neutral, slightly basic and slightly acidic media, but not at all the mono-charged cations K⁺, Na⁺, Cs⁺, Rb⁺, and NH₄⁺. The voltammetric processes of the synthesized O-demethylated 1,4-benzoquinone show clear sensitivity to the concentration of the Ca²⁺ Sr²⁺ Ba²⁺ and to smaller extent to Mg²⁺ cations. Increasing of the concentration of these earth-alkaline cations caused shifting of the mid-peak potentials (Δ*E*ₚ) of the redox process of synthesized O-demethylated benzoquinones towards more positive values. The mid-peak potentials of voltammetric curves representing the redox process of the O-demethylated benzoquinone were a linear function of the logarithm of the earth-alkaline cations concentration, with slope of those dependence having thermodynamic (Nernstian) values ranging between 55 mV and 62 mV/log[*c*(earth-alkaline cations²⁺)]. This finding implies that stochiometric complexes of type 1:2 between the reduced O-demethylated 1,4-benzoquinones and the earth-alkaline cations are formed. The intercepts of the dependence Δ*E*ₚ *_{VS}.* log[*C*(earth-alkaline cations²⁺)] hide the value of the stability constant of the complexes K_{stab}, which were estimated to be 1.95 x 10⁹ mol⁻¹ I³ for Ca²⁺, *K*_{stab} = 6.95 x 10⁷ mol⁻¹ I³ for Ba²⁺ and *K*_{stab} = 1.45 x 10⁶ mol⁻¹ I³ for Sr²⁺.

However, the stability constants between the electrochemically reduced O-demethylated benzoquinone ligands and the Mg²⁺ ions were found to be much smaller. Fore example, it was estimated that the stability constant of O-demethylated 2,6-dimethoxy 1,4-benzoquinone and the Mg²⁺ ions was *K*_{stab} = 4.05 ×10⁴ mol⁻¹ 1³. This value is actually about five orders of magnitude smaller than the corresponding one for Ca²⁺ ions. The smaller values of the stability constant with Mg²⁺ ions are indeed, linked to the ionic radii of magnesium ions that are much smaller than Ca²⁺ and Ba²⁺ ions, and therefore they are much strongly hydrated with the water molecules. The findings that the stability constant of the newly synthesized ligand with Ca²⁺ and Mg²⁺ ions are quite different is of exceptional importance, since they suggest that the electrochemically reduced forms of the O-demethylated 1,4-benzoquinones can be seen as selective ligands for Ca²⁺ and Mg²⁺ ions. This is very important because there is always a strong need to separate Ca²⁺ and Mg²⁺ ions that usually have quite different biological functions. It is worth to emphasize that identical values for the stability constants have been obtained for the complexes between the electrochemically reduced form of O-demethylated 2,5-dimethoxy 1,4-benzoquinone and the earth-alkaline cations Ca²⁺, Sr²⁺, Ba²⁺, and Mg²⁺. The stability constants of Ca²⁺, Sr²⁺, Ba²⁺ and Mg²⁺with electrochemically reduced form of O-demethylated Coenzyme Q0 are slightly different and they read: *K*_{stab} = 1.05 x 10⁸ mol⁻¹ I³ for Ca²⁺; *K*_{stab} = 4.50 x 10⁶ mol⁻¹ I³ for Ba²⁺; *K*_{stab} = 2.70 x 10⁶ mol⁻¹ I³ for Sr²⁺, and *K*_{stab} = 2.00 x 10⁴ mol⁻¹ I³ for Mg²⁺ ions. According to the foregoing discussion, the reaction of reduction and complex-formation of the O-demethylated 1,4-benzoquinone and Ca²⁺ (and Sr²⁺, Ba²⁺ or Mg²⁺) ions can be presented with following reaction scheme:

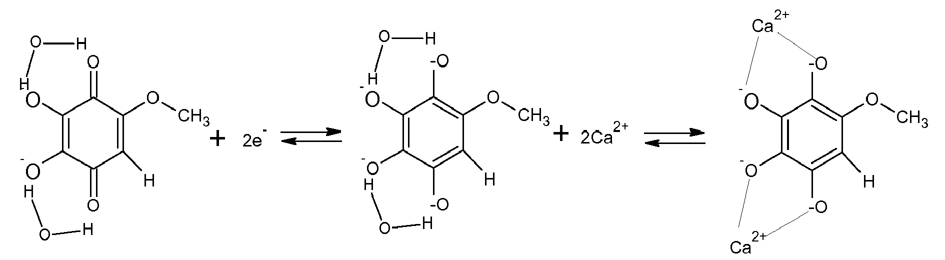

### Scheme 6: Reduction of the O-demethylatated form of 2,6-dimethoxy benzoquinone and its complexation process with Ca²⁺ ions.

It is well known that the complexing process of strongly hydrated earth-alkaline cations can be achieved almost exclusively by ligands that are highly negatively charged (EDTA⁴⁻, EGTA⁴⁻, or de-esterified forms of Fura 2 for example). In the reaction scheme 6, a highly negative charged structure is created upon the reduction of the oxidized form of O-demethylated benzoquinones. Namely, the electrochemically reduced forms of O-demethylated benzoquinones bear total charge of "4-". This amount of negative charge in the reduced forms of O-demethylated benzoquinones is, indeed, necessary for causing electrostatic interactions with the strongly hydrated hydrophilic earth-alkaline cations, thus creating rather stable complexes with them. It is worth to mention that such metal-complexating features are seen for the first time by the methoxy benzoquinones, and this can be of immense importance in understanding additional functions of these classes of quinones that are often present in various biological systems.

The invention will be further explained by the following Examples, which are however not to be construed so as to limit the invention.

### Examples

### Materials and Methods

Reagents, Solutions and Instrumentation: All the chemicals used were purchased from Sigma with 99 % or higher purity, if not otherwise stated. O-demethylated forms of 2,6-dimethoxy 1,4-benzoquinone, 2,5-dimethoxy 1,4-benzoquinone, 2,3-dimethoxy-6-methyl 1,4-benzoquinone (Coenzyme Q0) and Coenzyme Q1 (95 % purity) (see the structures in Scheme 1) were obtained by dissolving them in 1 mol/l NaOH solution. Approximately after 1 minute the color of the alkaline solutions of the respective quinone started to convert gradually from yellow via light-pink to dark-red. After defined time intervals aliquots of the alkaline stock solutions were transferred to the experimental cells. The concentration of the methoxy benzoquinones in the working cells was set to 1 mmol/l (in ESR experiments) or to 2 mmol/l for NMR experiments. The pH of the solutions was adjusted to the desired values by titration with 1 mol/l nitric acid and 0.5 mol/l NaOH. All solutions were prepared using deionized water.

Electrochemical Measurements: Cyclic voltammetry (CV) and square-wave voltammetric (SWV) electrochemical experiments were performed with a potentiostat model AUTOLAB PGSTAT12 (Eco Chemie, the Netherlands) in conventional three-electrode set-up. An Ag/AgCl (3 mol/l KCl) was used as a reference electrode, while the Platinum wire served as a counter electrode. The working electrode was a glassy carbon electrode (Metrohm) with diameter of 3 mm. The working electrode was cleaned immediately before each experiment by polishing it with Aluminum powder for 30 s, and rinsing with deionized water. All experiments were performed at room temperature (22 ± 2 °C).

UV-VIS Measurements: For the spectrophotometric experiments, we used UV/VIS Spectrophotometer Model *Ultrospec 2110 pro.* The measurements were performed in the spectral range from 200 to 900 nm. The behavior of the quinones in different solvents was followed in the kinetic mode.

EPR and NMR Measurements: The EPR experiments were performed with a Bruker spectrometer (ESP300e) equipped with a TMH cavity, in which the flat cell was placed immediately after filling with the reaction solution (120 µl). The modulation amplitudes varied between 10 mG and 0.2 G depending on the sample. The microwave power was set to 0.2 or 0.63 mW. Spectra were recorded with scan times of 10, 50 or 90 s and stored consecutively to monitor the kinetic behavior of the solution.

For the NMR experiments a 400 MHz Bruker Avance instrument was used to record standard 1D proton spectra. The solutions were prepared in D₂O and titrated to pD = 7.00 after defined reaction times in 1 mol/l NaOD. The samples (600 µl) were filled in precision quartz tubes for measurement at 290 K.

### Example I Coenzyme Q1.

Example I.1: A cyclic voltammogram of Coenzyme Q1 that was dissolved directly in neutral media was determined (Fig. 1). A single voltammetric process exists in neutral pH's, with a mid-peak potential of -0.36 V vs. Ag/AgCl.

Example 1.2: The insensitivity of the voltammetric process of native Coenzyme Q1 to calcium ions was determined (Fig. 2). An increase of the calcium ions concentration up to 100 mmol/l did not cause any changes in the mid-peak potential of the voltammetric process of CoQ1, which is an indicator that native CoQ1 structure can not bind calcium ions.

Example 1.3: The color of a solution of native Coenzyme Q1 directly dissolved in neutral aqueous media was compared with the color of a solution of Coenzyme Q1 that was initially dissolved in NaOH for 60 minutes, and retitrated afterwards to pH of 7.00. While the neutral solution of native CoQ1 is yellowish colored, the solution color of O-demethylated CoQ1 is intensively violet. This example shows us that two different forms of CoQ1 are present in both solutions.

Example 1.4: The changes in the voltammetric responses were determined that happened when CoQ1 is initially dissolved in strong alkaline solutions. In this case, two reversible redox processes are observed, whose intensities are significantly affected by the time. While the height of the first pair of peaks at more positive potentials decreases with the time, the intensity of the second pair of peaks at more negative potentials increases concomitantly (Fig. 3a-c). After period of 8 h in 1 mol/l NaOH the first voltammetric redox process of (it originates of native CoQ1) diminished significantly, while the second redox process at more negative potentials (it originates from the product formed in the reaction between native CoQ1 and NaOH) showed pronounced rising (Fig. 3d). These experiments implicate that after period of about 8 h, the reaction between CoQ1 and NaOH is significantly shifted toward the created product, and the synthesized product is predominant fraction in the solution.

Example I.5: The UV-VIS spectrum of Coenzyme Q1 that is obtained by its direct dissolution in water (pH of 7.00) was determined (Fig. 4). In this case, the UV-VIS spectrum consists of an absorption band located at about 280 nm, which is due to the absorption of both C=O carbonyl groups in the CoQ1 structure.

Example I.6: UV-VIS spectrophotometric kinetic measurements of CoQ1 dissolved in 1 mol/l NaOH were performed (Fig. 5). In this case, evident changes in the absorption bands have been observed with the time, signifying formation of a new compound. The initial UV-VIS spectrum of CoQ1 dissolved in 1 mol/l NaOH (after 5 min) consist of 2 absorption bands located at around 280 nm and around 325 nm, alongside to the band located at about 220 nm that is due to the absorption of the aromatic ring. The band at 280 nm is due to the absorption of C=Ogroup of the native form of CoQ1 (see Fig. 4), while the band at 325 nm is due to the absorption of the product created via the reaction of CoQ1 and NaOH. While the intensity of the first absorption band at 280 nm decreases with the time, the intensity of the second band at 325 continuously augments for same extent. After a period of about 4 h the chemical equilibrium is significantly shifted toward the product of the chemical reaction, yielding mainly a broad intensive absorption peak at 325 nm (curve 210 min in Fig. 5).

Example I.7: The cyclic voltammograms of the native (yellow) form of CoQ1 (Fig. 6, curve 1) was compared with that of the (violet colored) form of CoQ1, obtained after reaction of native CoQ1 with NaOH (Fig. 6, curve 2). In both cases, the final pH of water solutions was adjusted to pH = 7.40. Evidently, both voltammograms differ fundamentally, that is, they originate from two different structures of CoQ1. While the mid-peak potential of the redox process of native CoQ1 (curve 1) is -0.36 V vs. Ag/AgCl, the mid-peak potential of the synthesized O-demethylated CoQ1 (curve 2) is -0.70 V. vs. Ag/AgCl. This example clearly shows that the reduced form of the synthesized purple O-demethylated CoQ1 is for -340 mV much stronger antioxidant than its parent compound.

Example I. 8: The top panel of Fig. 7 shows the radical produced by reduction of CoQ1 with sodium borhydrid in neutral solution. The lower spectrum is the simulation (sim). The bottom panel of Fig. 7 presents the time evolution of the same spectrum in alkaline medium.

Example I.9: The nuclear magnetic resonance (NMR) spectra of Coenzyme Q1 initially dissolved in 1 mol/l NaOD solution, and retitrated after different reaction times with DNO₃ to pD of 7.00 was established (Fig. 8).

Example I.10: The ratio of the NMR signal intensities of created methanol vs. that of the methoxy groups in the structure of Coenzyme Q1 was determined (Fig. 9). This ratio increases linearly from 0 to 1.2, after reaction times of CoQ1 and NaOH ranging from 5 to 400 min. This is a clear indicator that the methanol is produced by cleaving of the two methoxy groups of CoQ1 with NaOH.

Example I.11: The voltammetric behavior of CoQ1 was determined, which was initially dissolved for certain time in strong alkaline media (15 min to 8 h), retitrated afterwards to different pH's. In solutions having pH from 3.0 to 12.0, two redox processes at the cyclic voltammograms exist (Fig. 10). The first redox process (at more positive potentials) originates from the native (parent) form of CoQ1, and its mid-peak potential is mainly a proton sensitive item. The mid-peak potential of the second electrode process (at more negative potentials) is insensitive to proton concentration, in pH regions from 4.5 to 12. The origin of the redox process at very negative potentials is the reduction-oxidation of the negatively charged O-demethylated CoQ1 form. The highly negative potential needed to reduce the synthesized O-demethylated CoQ1 is, indeed, consistent with the huge electronic density present in its structure. The insensitivity of the second redox process to the H⁺ concentration implies that the contributors in this second redox process are strongly stabilized via strong hydrogen bonds with the solvent molecules. Next to the high potential for hydrogen bonding, the effect of charge delocalization, which is a common feature of conjugated resonance aromatic structures, can also play significant role in the electrochemical features of the O-demethylated CoQ1.

Example I.12: The sensitivity of voltammetric process of the synthesized O-demethylated CoQ1 to the concentration of the Ca²⁺ cations was determined (Fig. 11). In this case, the voltammetric responses of the O-demethylated CoQ1 structure are highly sensitive to calcium concentration, producing a shift of the voltammetric response of O-demethylated CoQ1 towards more positive potentials by increasing the calcium concentration. The mid-peak potentials (Δ*E*ₚ,_{mid}) of voltammograms at Fig. 11 were linear function of the logarithm of the Ca²⁺ concentration, with slope of that dependence having a thermodynamic value of 61 mV/ log[*C*(Ca2⁺)]. This finding implies that a stochiometric complex of type 1:2 between the electrochemically reduced O-demethylated CoQ1 and Ca²⁺ ions was formed. The stability constant of the complex was estimated to be 6.60 x 10⁶ mol⁻¹ 1³.

### Example II. 2,6-dimethoxy 1,4-benzoquinone.

Example II.1: The color of the solution, when 1 mmol/l 2,6- dimethoxy 1,4-benzoquinone is directly dissolved in neutral aqueous media. An intensively yellow-colored solution is observed in the pH regions from 2.0 to 9.0. The same color was observed for the other two dimethoxy benzoquinones, i.e. 2,5-dimethoxy 1,4-benzoquinone and Coenzyme Q0.

Example II.2: The sensitivity of the redox process of the native (yellow form) 2,6-dimethoxy 1,4-benzoquinone to the proton concentration was determined (Fig.12). The mid-peak potentials of the cyclic voltammograms shift for 60 mV/pH in positive direction by a decade increasing of the concentration of the H⁺ ions in the solution. This situation corresponds to the 2e-/2H⁺ reduction of 2,6-dimethoxy 1,4-benzoquinone to 2,6-dimethoxy 1,4-hydroquinone, and it can be represented by following reaction scheme which shows the redox process of the native 2,6-dimethoxy 1,4-benzoquinone in aqueous media having pH from 1.0 to 9.0:

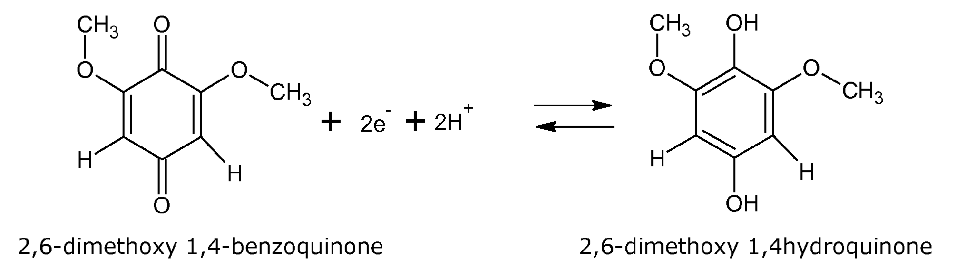

Example II.3: The color of the solutions of 1 mmol/l; 2,6-dimethoxy 1,4-benzoquinone from Example II.1, and the solution color in pH of 7.0, but with 2,6-dimethoxy 1,4-benzoquinone initially dissolved in 1 mol/l water solution of NaOH for 90 minutes was compared. While the color of the water solution of 2,6-dimethoxy 1,4-benzoquinone directly dissolved in neutral media is intensively yellow, the color of the solution of 2,6-dimethoxy 1,4-benzoquinone that is initially dissolved in aqueous alkaline solutions for certain time (and retitrated afterwards to pH of 7.00) is intensively red. This simple comparison of the solution colors clearly illustrates that the two forms of existing in neutral media must have quite different electronic structure. Moreover, the product created in such scenario shows long-lasting stability, if the initial alkaline solution in which 2,6-dimethoxy 1,4-benzoquinone is dissolved is retitrated to neutral pH's after no longer than 12 h.

Example II.4: The method for synthesizing of the red form of 2,6-dimethoxy 1,4-benzoquinone via its reaction with NaOH is given. Principally, we put 3.36 mg of solid 2,6-dimethoxy 1,4-benzoquinone in 10 ml water alkaline solution of 1 mol/l NaOH, and we let the reaction to proceed at room temperature, until the color of the solution gets intensively dark red. The changes of the color solution with the time give insights about the chemical reaction taking place in the system. When 1 mmol/l 2,6-dimethoxy 1,4-benzoquinone is put in 1 mol/l alkaline water solution of NaOH, the color of the solution starts to change from yellow via pink and reddish, while getting a dark red color after couple of h. These color changes are due to the reaction of the 2,6-dimethoxy 1,4-benzoquinone with NaOH that actually leads to production of a new benzoquinone structure. The reaction product is stable if the re-titration of the alkaline solution is made up to 12 h after initiation of the reaction. Otherwise, due to the complex radical-radical interactions, other compounds can be formed that have much different features than the product presented in the second part of Example II.3.

Example II.5: The EPR spectra shown in Fig. 13 demonstrate the differences of signals obtained for 2,6-dimethoxy 1,4-benzoquinone in neutral and strongly basic conditions. At neutral pH the signal is produced by chemical reduction of the compound (with sodium borhydride as reducing agent), whereas in basic conditions the radicals are formed by nucleophilic attack of the hydroxyl anion (without sodium borhydride). These spectra can only be explained by the cleavage of the methyl group in one methoxy-substituent. The absence of the small splitting in deuterated solvent (Fig. 13C) proves that a hydoxyl group is attached to the ring system. The Ratio of the relative integrals from the NMR signals of methanol vs. the NMR signal of methoxy groups as a function of time is shown in Fig. 14.

Example II.6: The changes in the voltammetric responses of 2,6-dimethoxy 1,4-benzoquinone, when it is dissolved in 0.1 mol/l alkaline water solution of NaOH is shown in Fig. 15. The voltammetric peak at more positive potentials (Peak I) originates from the redox process of the native (yellow) form of 2,6-dimethoxy 1,4-benzoquinone. The intensity of this peak decreases by the time, which implies that the concentration of the yellow form of 2,6-dimethoxy 1,4-benzoquinone is diminishing with the time. On the other hand, a new process appears at more negative potentials (Peak II). The origin of this redox process (Peak II) is the reduction-oxidation of the O-demethylated product synthesized via the chemical reaction between 2,6-dimethoxy 1,4-benzoquinone and NaOH (the red form). The intensity of the voltammetric peaks representing this second redox process increases with the time, which means that the concentration of the formed product also rises with the time.

Example II.7: The changes in the UV-VIS spectrum of 2,6-dimethoxy 1,4-benzoquinone when it is dissolved in alkaline water solution of 1 mol/l NaOH are shown in Fig. 16. When 2,6-dimethoxy 1,4-benzoquinone from Example 1 is dissolved directly in neutral media, the absorption spectrum of 2,6-dimethoxy benzoquinone consists of one main absorption band located at 250 nm, which is due to absorption of C=O groups in positions 1 and 4 from the structure of 2,6-dimethoxy 1,4-benzoquinone (Fig. 16A). However, when 2,6-dimethoxy 1,4-benzoquinone is dissolved initially in 1 mol/l alkaline water solution of NaOH, then a new absorption band appears in the UV-VIS spectrum, located at about 300 nm. While the intensity of the absorption band at 250 nm decreases with the time, the intensity of the new absorption band at 300 nm increases for same extent as the first one band decreases (Fig. 16B). After period of 24 h, the only existing absorption band is that at 300 nm (Fig. 16C). These experiments clearly illustrate that the yellow form of 2,6-dimethoxy 1,4-benzoquinone reacts with NaOH, and it is converted to a new structure.

Example II.8: The quite different lipophilic properties between the yellow form of 2,6-dimethoxy 1,4-benzoquinone from Example II.1, and the red synthesized form from Example II.4 were compared. When 0.5 ml solution of the native (yellow form) 2,6-dimethoxy 1,4-benzoquinone (c = 1 mmol/l) is mixed and shake with 0.5 ml of water-immiscible (colorless) lipophilic organic solvent 1,2-dichlorethan, we observe that 2,6-dimethoxy 1,4-benzoquinone distributes very quickly to the organic solvent. The colorless 1,2-dichlorethan organic phase becomes yellow colored. This finding suggests that the yellow form of 2,6-dimethoxy 1,4-benzoquinone from Example II.1 has amphiphilic properties, i.e. it is soluble to some extent in water, but it is nicely soluble in organic solvents. Oppositely, when the red benzoquinone form synthesized in Example II.4 is mixed and shake with 1,2-dichlorethan, there is no partition at all to the organic solvent. This finding implicates that the red O-demethylated benzoquinone form from Example 4 features a quite hydrophilic nature, which means that this form is very polar and does not dissolve at all in organic lipophilic solvents.

Example II.9: The pH insensitivity of the redox process of synthesized O-demethylated benzoquinone from Example II.4, in pH regions between 4.5 < pH < 13.0 is shown in Fig. 17. Initially, a 1 mmol/l 2,6-dimethoxy 1,4-benzoquinone was let to react with 0.1 mol/l NaOH solution for 60 min. Subsequently, the pH of the solution was adjusted to the desired values by titration with 2 mol/l HN0₃, and cyclic voltammograms were recorded afterwards. In aqueous solutions having pH > 4.5, we observed two quasireversible redox processes. The first redox process (i.e. Peak I at more positive potentials) is from the native (yellow form) of 2,6-dimethoxy 1,4-benzoquinone. The mid-peak potential of this redox process is proton sensitive item in a large region of pH's, and the behavior of this redox process is described in Example 2. However, the mid-peak potential of the second voltammetric process (Peak II at more negative potentials) is completely insensitive to proton concentration over a pH's range between 4.5 < pH < 13.0. The origin of this redox process (Peak II) is the reduction-oxidation of the synthesized benzoquinone product created via the chemical reaction between 2,6-dimethoxy and OH- in strongly alkaline solutions as described in Example 4. The highly negative potential (about -0.7 V) needed to reduce the synthesized compound from Examples 4 and 5 is, indeed, consistent with the huge electronic density present in its structure. Moreover, the high insensitivity of the second redox process to the proton concentration implies that the electrochemical products of negatively charged benzoquinone derivative must be stabilized via strong hydrogen bonds with the water molecules. This example shows that the redox chemistry of the native 2,6-dimethoxy 1,4-benzoquinone from Example II.1, and the synthesized O-demethylated (red form) benzoquinone from Example II.4 is completely opposite in respect to proton sensitivity. If we make retitration of the initial alkaline solution in which 2,6-dimethoxy 1,4-benzoquinone is dissolved for no longer than 12 hours, then we get a highly stable form of the formed O-demethylated benzoquinone product.

Example II.10: The electrochemically reduced form of the synthesized O-demethylated benzoquinone from Example 4 (retitrated to pH of 7.40 after being 60 min in contact with NaOH) possesses intrinsic properties to complexate earth-alkaline cations (Ca²⁺, Ba²⁺, Sr²⁺ and to much smaller extent Mg²⁺) in neutral, slightly basic and slightly acidic media (Fig. 18). Initially, a 1 mmol/l 2,6-dimethoxy 1,4-benzoquinone was let to react with 0.1 mol/l NaOH solution for 60 min. Subsequently, the pH of the solution was adjusted to 7.40 by making titration with 2 mol/l HN0₃. In addition, square-wave voltammograms were recorded, in experiments where the concentration of the particular earth-alkaline cation in the electrochemical cell has been consecutively increased. The voltammetric process of the synthesized red form of O-demethylated benzoquinone (the process happening at more negative potentials) showed clear sensitivity to the concentration of the Ca²⁺ Sr²⁺ Ba²⁺ and to smaller extent to Mg²⁺ cations. Increasing of the concentration of these earth-alkaline cations caused shifting of the mid-peak potentials (Δ*E*ₚ) of the redox process of synthesized O-demethylated benzoquinone towards more positive values. The mid-peak potentials of voltammetric curves representing the redox process of the synthesized O-demethylated benzoquinone were a linear function of the logarithm of the earth-alkaline cations concentration. The slopes of those dependence have thermodynamic (Nernstian) values ranged between 55 mV to 62 mV/log[*c*(Earth-alkaline Cations²⁺)], suggesting that stochiometric complexes of type 1:2 between the reduced O-demethylated benzoquinone and the earth-alkaline cations were formed (Galus, Z., Fundamentals of Electrochemical Analysis, New York and Warsaw: Ellis Horwood and Polish Scientific Publishers PWN (1994)). From the intercepts of the dependence Δ*E*ₚ *VS*. log[*C*(Earth-alkaline Cations²⁺)] (Galus, Z., Fundamentals of Electrochemical Analysis, New York and Warsaw: Ellis Horwood and Polish Scientific Publishers PWN (1994)) we determined the stability constant of the complexes *K*_{stab}, which were estimated to be 1.95 x 10⁹ mol⁻¹ I³ for Ca²⁺; *K*_{stab} = 1.45 x 10⁶ mol⁻¹ I³ for Sr²⁺; and *K*_{stab} = 6.95 x 10⁷ mol⁻¹ I³ for Ba²⁺. However, the stability constant between the reduced O-demethylated benzoquinone and Mg²⁺ ions was found to be much smaller, and it was estimated to be *K*_{stab} = 4.05 x 10⁴ mol⁻¹ I³ that is about five orders of magnitude smaller than that of Ca²⁺. This finding shows that the synthesized O-demethylated benzoquinone from Example II.4 is a very selective ligand for Ca²⁺ and Mg²⁺ ions.

Example II.11: In the Examples II.1 to II.10, mainly the features of the compound 2,6-dimethoxy benzoquinone are described. However, everything that has been described and explained in Examples II.1 to II.10 holds virtually in identical manner for the compounds 2,5-dimethoxy 1,4-benzoquinone and 2,3-dimethoxy 6-methyl benzoquinone (Coenzyme Q0). The method for the synthesis of the O-demethylated forms of these compounds is the same as that described in Example II.4. In this example we only illustrate the voltammetric responses of synthesized O-demethylated red forms of 2,5-dimethoxy 1,4-benzoquinone and Coenzyme Q0 showing their sensitivity to Ca²⁺ ions. It is worth to emphasize that identical values for the stability constants as those reported in Example II.10 have been obtained for the complexes between the electrochemically reduced form of O-demethylated 2,5-dimethoxy 1,4-benzoquinone and the earth-alkaline cations Ca²⁺, Sr²⁺, Ba²⁺, and Mg²⁺. The stability constants of Ca²⁺, Sr²⁺, Ba²⁺ and Mg²⁺ with electrochemically reduced form of O-demethylated Coenzyme Q0 are slightly different and they read: *K*_{stab} = 1.05 x 10⁸ mol⁻¹ I³ for Ca²⁺; *K*_{stab} = 4.50 x 10⁶ mol⁻¹ I³ for Ba²⁺; *K*_{stab} = 2.70 x 10⁶ mol-¹ I³ for Sr²⁺; and *K*_{stab} = 2.00 x 10⁴ mol⁻¹ I³ for Mg²⁺ ions (Fig. 19).

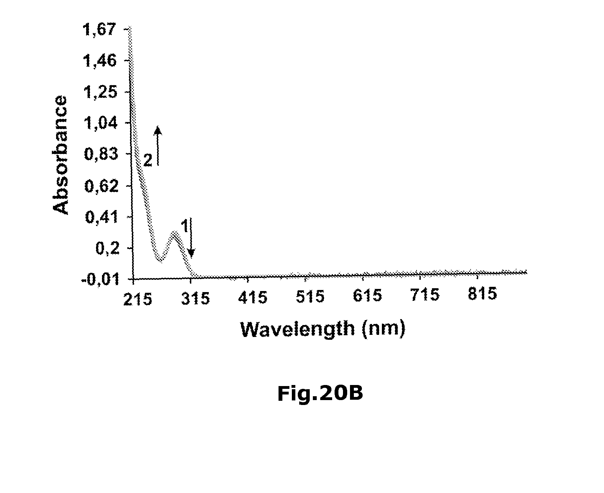

Example II.12 The superior anti-oxidative properties of the O-demethylated water soluble forms of hydrophilic benzoquiones synthesized from Examples II.4 and II.11 against the native dimethoxy 1,4-benzoquinones forms from Example II.1 is shown in Fig. 20. The voltammograms presented in Example II.9 to II.11 recorded in neutral media clearly show that the redox processes of the synthesized O-demethylated benzoquinone products take place at potentials that are more negative for -300 mV to -500 mV (Peak II) than those of the native (yellow) dimethoxy benzoquinones from Example 1 (Peak I). The high negative potentials of the redox process of synthesized O-demethylated benzoquinones (*E*_{p,mid} = -0.70 V to *E*_{p,mid} = -0.80 V) imply that the reduced forms of the O-demethylated forms from Examples II.4 and II.11 are much stronger antioxidants than the native dimethoxy benzoquinones from Example II.1. To prove this, additional UV-VIS experiments were performed, in which the kinetics of the reaction of the reduced 2,6-dimethoxy 1,4-benzoquinone from Example II.1 and the reduced form of the O-demethylated benzoquinone from Example II.4 with the oxygen in neutral media were followed. In these experiments, a 5 ml solution containing 1 mmol/l concentration of the yellow form from Example 1, or the red form from Example II.4 have been initially completely converted to its reduced form via reaction with a stochiometrical amount of sodium borhydride NaBH₄ (c(NaBH₄) = 10 mmol/l). The solution of the reduced form of the synthesized O-demethylated benzoquinone from Example II.4 is slightly yellowish, while the solution of the reduced form of the native 2,6-dimethoxy 1,4-benzoquinone from Example 1 is colorless. The obtained solutions were consequently diluted with water to get concentration of 50 µmol/l. Afterwards, 2 ml of that solution was placed into the cuvete, and their reaction with oxygen has been followed in kinetic mode. It was observed that the solution with the reduced form of O-demethylated benzoquinone from Example II.4 regained the color much faster than the native 2,6-dimethoxy 1,4-benzoquinone (Fig. 20). That means that the reaction between the reduced form of the synthesized O-demethylated benzoquinone from Example II.4 and oxygen is much faster than the corresponding reaction between the native 2,6-dimethoxy 1,4-benzoquinone and the oxygen. This finding implies that the reduced form of the O-demethylated benzoquinone from Example II.4 can act as much stronger antioxidant than the native 2,6-dimethoxy 1,4-benzoquinone from Example II.1.

## Claims

1. A di-, tri- or tetraoxy-para-benzoquinone compound having the formula (I) 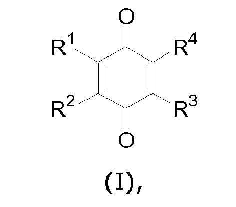 wherein at least two of R¹ to R⁴ are O- residues and the remaining are independently selected from H, alkyl, alkenyl and alkoxy, or, if adjacent, may form a carbocyclic or heterocyclic ring, which may be saturated, unsaturated or aromatic, together with the two carbon atoms of the benzoqinone ring, or a salt thereof.

2. The compound of claim 1 which is a dioxy-para-benzoquinone compound having a formula (IIa), (IIb) or (IIc) 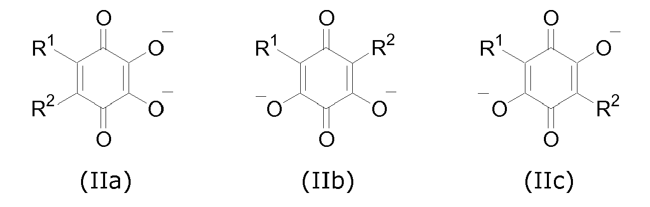 wherein R¹ and R² are independently selected from H, alkyl, alkenyl and alkoxy, or in formula (IIa) R¹ and R², together with the two carbon atoms of the benzoqinone ring, may form a carbocyclic or heterocyclic ring which may be saturated, unsaturated or aromatic, or a salt thereof, preferably the compound is a 2,3-dioxy-para-benzoquinone compound having a formula (IIa) or a salt thereof.

3. The compound of claim 1 or 2, wherein
(i) the remaining residues R¹ to R⁴ or R¹ and R² are independently selected from H, C₁₋₆alkyl, C₂₋₆alkenyl, and C₁₆alkoxy, preferably from H, C₁₋₄alkyl, C₂₋₄alkenyl, and methoxy; and/or
(ii) the cation forming the salt is selected from earth alkaline metal cations including Mg²⁺, Ca²⁺, Ba²⁺ and Sr²⁺ ions.

4. The compound of claim 2 or 3, which is a 2,3-dioxy-para-benzoquinone compound selected from 2,3-dioxy-6-methoxy-para-benzoquinone, 2,3-dioxy-6-methyl-parabenzoquinone (O-demethylated Coenzyme Q0), 2,3-dioxy-5-isoprenyl-6-methyl-para-benzoquinone (O-demethylated Coenzyme Q1), and salts thereof.

5. The compound of claim 4, wherein the stability constant (*K*_{stab}.) with earth-alkaline metals are as follows:
*K*_{stab}. = 1.95 x 10⁹ mol⁻¹ I³ for Ca²⁺; *K*_{stab}. = 6.95 x 10⁷ mol⁻¹ I³ for Ba²⁺; *K*_{stab}. = 1.45 x 10⁶ mol-¹ I³ for Sr²⁺; and *K*_{stab}. = 4.05 x 10⁴ mol⁻¹ I³ for Mg²⁺ i(for 2,3-dioxy-6-methoxy-para benzoquinone); and
*K*_{stab} = 1.05 x 10⁸ mol⁻¹ I³ for Ca²⁺; *K*_{stab} = 4.50 x 10⁶ mol^{-1 3} for Ba²⁺; *K*_{stab} = 2.70 x 10⁶ mol-¹ I³ for Sr²⁺; and *K*_{stab} = 2.00 x 10⁴ mol⁻¹ I³ for Mg²⁺ (for 2,3-dioxy-6-methyl parabenzoquinone).

6. The compound of any one of claims 1 to 5, which
(i) is highly soluble in water, preferably more than 0.05 mol/l, but highly insoluble in lipophilic organic solvents including 1,2-dichlorethan, nitrobenzene, and nitrophenyl octyl ether and hardly soluble in alcohols;
(ii) shows long-lasting stability, when retitrated to neutral within 12 h after its production;
(iii) forms an at least -300 mV stronger antioxidant than the corresponding reduced form in neutral media;
(iv) is insensitive to pH in the regions 4.5 < pH < 13.0;
(v) is unable to form complexes with mono-charged alkaline cations including Li⁺, Na⁺, K⁺, Rb⁺, Cs⁺ and NH₄⁺; and/or
(vi) is a selective ligand for Ca²⁺and Mg²⁺ ions in neutral media.

7. A method for preparing the di-, tri- or tetraoxy-para-benzoquinone compound having the formula (I), (IIa), (IIb) or (IIc) as defined in any one of claims 1 to 6, which comprises reacting a starting benzoquinone derivative of the formula (I) 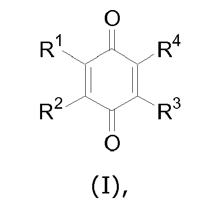 wherein two out of R¹ to R⁴ are independently selected from H, alkyl, alkenyl and alkoxy, or, if adjacent, may form a carbocyclic or heterocyclic ring, which may be saturated, unsaturated or aromatic, together with the two carbon atoms of the benzoqinone ring, and the two others are independently selected from H and alkoxy, with a concentrated aqueous alkaline solution.

8. The method of claim 7, wherein the prepared benzoquinone is a compound of formula (IIa) as defined in claim 2, preferably is a compound as defined in claim 4, wherein the starting benzoquinone of formula (I) is most preferably selected from 2,6-dimethoxy benzoquinone, 2,5-dimethoxy benzoquinone, 2,3-dimethoxy-6-methyl benzoquinone (Coenzyme Q0) and Coenzyme Q1

9. The method of claim 7 or 8, wherein
(i) the concentrated aqueous alkaline solution is selected from an aqueous NaOH, KOH and LiOH solution; and/or
(ii) the reaction is performed by reaction at pH 11 to 13, at a temperature of 15 to 30 °C, preferably at 20 to 25 °C, for 20 min to 24 h, preferably 30 min to 12 h.

10. The method of any one of claims 7 to 9, which further comprises retitration of the O-demethylated product to neutral, preferably at least 30 minutes after the O-demethylation reaction.

11. A composition, reduction agent or stabilizer comprising a compound of any one of claims 1 to 6 or a mixture thereof.

12. Use of the compound of any one of claims 1 to 6 for preparing a medicament having antioxidant properties.

13. Use of the compound of claims 1 to 6 as antioxidant, anti-ageing agent, in organic synthesis, for metal complexations, metal separations, potentiometric titrations, interfacial catalysis, and ion-transfer reactions across biological membranes.
